# FASCICULE DE BREVET EUROPEEN

(11) **EP 3 107 895 B1**
(45) Date de publication et mention de la délivrance du brevet: **27.12.2017**
(21) Numéro de dépôt: 15709268.5
(22) Date de dépôt: 17.02.2015
(51) Int. Cl.: C07C 319/14, C07C 323/52, C09K 15/14, C08K 5/375, C09J 11/06

(54) **DÉRIVÉS PHÉNOLIQUES SOUFRÉS**
SCHWEFELHALTIGE PHENOLDERIVATE
SULFUR-CONTAINING PHENOL DERIVATIVES

(30) Priorité: 20.02.2014 FR 1451369
(43) Date de publication de la demande: 28.12.2016
(73) Titulaire: Arkema France, 92700 Colombes (FR)
(72) Inventeur: SAINT-LOUIS-AUGUSTIN, Pascal, F-64140 Billere (FR); CAZAUX, Jean-Benoît, F-64140 Billere (FR)
(86) Numéro de dépôt international: PCT/FR2015/050385
(87) Numéro de publication internationale: WO 2015/124862

(56) Documents cités:
- US-A- 4 759 862
- PROSENKO A E ET AL: "Synthesis and investigation of antioxidant properties of alkylated hydroxybenzyl dodecyl sulfides", PETROLEUM CHEMISTRY, NAUKA/INTERPERIODICA, MO, vol. 46, no. 4, 1 juillet 2006 (2006-07-01), pages 283-288, XP019414296, ISSN: 1555-6239, DOI: 10.1134/S0965544106040116

## Description

La présente invention concerne un nouveau procédé de préparation de composés phénoliques soufrés, et de nouveaux dérivés du phénol substitués par au moins un radical comportant un atome de soufre. Ces composés phénoliques soufrés trouvent des applications dans de nombreux domaines de l'industrie, notamment de l'industrie chimique, et sont, par exemple, particulièrement adaptés en tant qu'agents anti-oxydants, stabilisants U.V., stabilisants thermiques, et autres.

La littérature fournit déjà de nombreux exemples de dérivés phénoliques, en particulier de dérivés phénoliques soufrés. L'industrie recherche cependant, de manière continue, des composés toujours plus performants, moins toxiques, moins odorants, plus respectueux de l'environnement, plus faciles à préparer à moindres coûts, pour ne citer que les principales raisons.

En ce qui concerne les anti-oxydants, la demande internationale WO1997014678 divulgue des composés comportant des groupements phénol de formule (A) suivante : 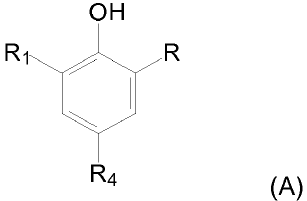 dans laquelle R, R₁ et/ou R₄ peuvent représenter un groupement de type -CH₂-S-(CH₂)ₙ-ester ou -CH₂-S-(CH₂)ₙ-amide, et où n est égal à 1 ou 2.

Le brevet US4759862 décrit la préparation de phénols de formule (B) suivante, qui sont utilisés comme stabilisants de polymères et d'huiles : 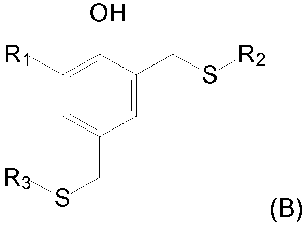 formule (B) dans laquelle R₂ et R₃ peuvent représenter chacun un groupement de type -CH₂-S-(CH₂)₁₋₃-W, où W représente un groupement ester ou amide.

Le brevet US4857572 décrit la préparation de stabilisants possédant la structure de phénols substitués de formule générale (C) suivante :

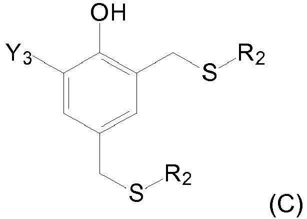

Selon cette formule générale, les phénols substitués peuvent comporter des groupements de type -CH₂-S-CH₂-C(O)OCH₃, et/ou de type alkyle en C₁-C₁₈ éventuellement alkyle substitué par -COOR₅.

Le brevet US4091037 décrit la préparation d'alkylthiométhylphénols dont la structure générale (D) est représentée ci-dessous : 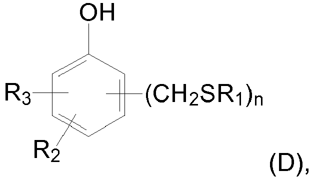 Structure générale (D) dans laquelle le groupement R₁ ne peut pas comporter de groupement ester.

Le brevet US4874885 décrit un procédé de préparation de mercaptomethylphénols de structure générale (E) suivante : 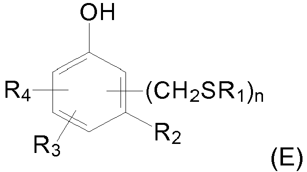 où le groupement R₁ peut représenter un groupement (C₁-C₂₀)-alkyle ou un groupement (C₁-C₄)-alkylène-C(O)OR₅.

Le brevet US3227677 revendique la préparation de bis(hydrocarboxy-carbonylalkylthioalkyl)phénols de formule générale (F) suivante : 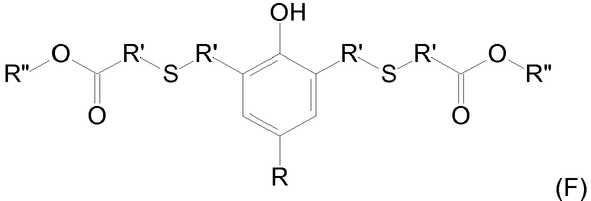 où R représente l'hydrogène ou un alkyle en C₁-C₇, R' représente un alkylène en C₁-C₇ et R" est un alkyle, aryle et/ou cycloalkyle en C₄-C₁₈.

Cette formule générale montre que sont seuls décrits des composés comportant deux groupements -R'-S-R'-C(O)OR", dans lesquels R' représente un groupement alkylène comportant de 1 à 7 atomes de carbone et R" représente un groupement alkyle, aryle et/ou cycloalkyle de 4 à 18 atomes de carbone.

La demande de brevet US20080081929 décrit une méthode de préparation de thiométhylphénols de structure (G) représentée ci-dessous : 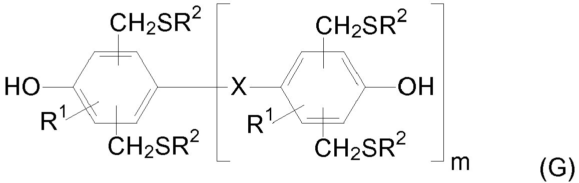 à partir des précurseurs phénoliques correspondants et des mercaptans de formule R²SH, où R₂ représente un radical alkyle, éventuellement comprenant un radical aryle, linéaire ou ramifié, en C₁-C₁₆.

Le brevet DE19822251 divulgue des composés de structure (H) : 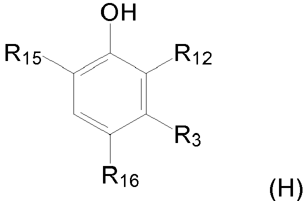 dans laquelle au moins un des R₃, R₁₂, R₁₅ et R₁₆ représente un groupe -CₙH₂ₙ-S-CₘCH₂ₘ-COOR₁₃, où R₁₃ représente hydrogène ou alkyle, n représente 0, 1 ou 2 et m représente 1 ou 2.

Le brevet US6028131 décrit la préparation d'antioxydants répondant à la formule (I) suivante : 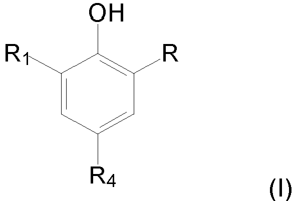 où R peut représenter un groupement -CH₂-A-R₂, dans lequel A représente S ou SO et R₂ peut être un groupe -(CH₂)ₘ-C(O)OR₅, où m est égal à 1 ou 2 et R₅ est un radial C₁-C₁₈ alkyle ; les autres radicaux R₁ et R₄ ne comportant pas d'atome de soufre. PETROLEUM CHEMISTRY, 46(4), 2006, 283-288 décrit un procédé dans lequel un dérivé phénolique aminé réagit avec un alkylthiol.

Un premier objectif de la présente invention consiste à proposer un nouveau procédé de préparation de tels composés phénoliques soufrés, ledit procédé étant plus simple à mettre en oeuvre, notamment sur le plan industriel, plus respectueux de l'environnement, mettant en oeuvre des composés moins toxiques que ceux utilisés dans les procédés de préparation de composés phénoliques soufrés connus de l'art antérieur, et plus généralement mettant en oeuvre des produits de départ d'origine bio-ressourcée, en particulier des produits de départ issus de matières premières d'origine végétale ou animale.

Un des avantages du procédé de préparation selon l'invention réside dans le fait que les composés phénoliques soufrés obtenues ne présentent que peu ou pas d'odeurs, en particulier d'odeurs désagréables, telles que celles que l'on peut ressentir avec certains composés phénoliques soufrés préparés selon les procédés de préparation connus et qui conduisent à des composés phénoliques soufrés contenant des traces de produits de départ nauséabonds qui n'ont pas réagi, tels les mercaptans, en particulier certains n-alkylmercaptans.

Le procédé de préparation selon l'invention permet en outre d'atteindre de nouveaux composés phénoliques soufrés préparés au moins en partie à partir de matières premières renouvelables, et plus particulièrement à partir d'acides gras d'origine végétale ou animale. La présente invention offre ainsi à l'homme du métier de nouveaux composés phénoliques soufrés qui sont moins toxiques, plus respectueux de l'environnement, et ne présentant que peu ou pas d'odeurs, en particulier d'odeurs désagréables, telles que celles que l'on peut ressentir avec certains composés phénoliques soufrés connus et qui comportent des traces de produits de départ nauséabonds qui n'ont pas réagi, tels les mercaptans, en particulier certains n-alkylmercaptans.

Comme les composés phénoliques soufrés connus de l'art antérieur, les nouveaux composés phénoliques soufrés de l'invention peuvent par exemple, et de manière non limitative, être utilisés par exemple en tant qu'agents anti-oxydants, stabilisants U.V., stabilisants thermiques, dans de nombreuses applications, et particulièrement dans la préparation de matières plastiques, fibres synthétiques, élastomères, adhésifs, additifs lubrifiants, etc....

D'autres objectifs encore apparaîtront dans la description qui suit. Les objectifs précités sont atteints en totalité ou au moins en partie, grâce aux composés de la présente invention.

Ainsi, et selon un premier aspect, la présente invention a pour objet un procédé de préparation, à partir de matières premières d'origine renouvelable, de composés phénoliques soufrés répondant à la formule (1) suivante : 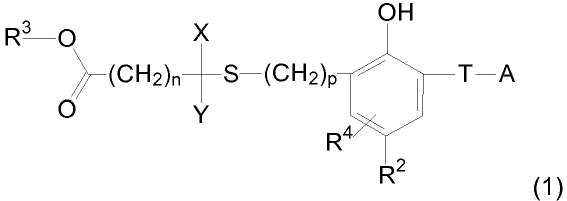 dans laquelle :
- A représente un radical R¹ ou un radical de formule A^{m} : 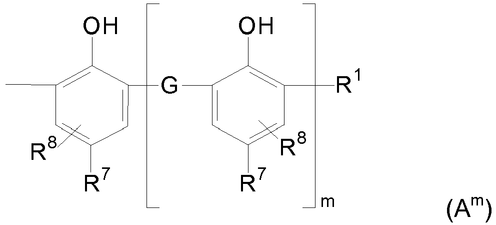
- R¹ est choisi parmi l'atome d'hydrogène, un groupement hydrocarboné linéaire ou ramifié comprenant de 1 à 20 atomes de carbone, et un groupement - (CH₂)ₚ-S-C(XY)-(CH₂)ₙ-C(O)OR³ ;
- R² est choisi parmi un groupement hydrocarboné linéaire ou ramifié comprenant de 1 à 20 atomes de carbone, et un groupement -(CH₂)ₚ-S-C(XY)-(CH₂)ₙ-C(O)OR³ ;
- R³ représente un groupement hydrocarboné linéaire ou ramifié comprenant de 1 à 20 atomes de carbone ;
- R⁴, R⁷ et R⁸, identiques ou différents, sont choisis, indépendamment l'un de l'autre, parmi l'atome d'hydrogène, un groupement hydrocarboné linéaire ou ramifié comprenant de 1 à 20 atomes de carbone, et un groupement -(CH₂)ₚ-S-C(XY)-(CH₂)ₙ-C(O)OR³ ;
- G est choisi parmi -S_{c}-, -(CH₂)ₐ-, -C(CH₃)₂-, -[S(O)_{b}]_{c}- et -W-, où W est un groupement aromatique, éventuellement substitué par un ou plusieurs groupes alkyle ;
- T est choisi parmi une liaison simple, -Sᵥ-, -(CH₂)ₜ-, -C(CH₃)₂-, et -[S(O)ᵤ]ᵥ- ;
- X et Y, indépendamment l'un de l'autre, représentent chacun un radical choisi parmi l'atome d'hydrogène et les groupements hydrocarbonés, linéaires ou ramifiés, comportant de 1 à 20 atomes de carbone, et éventuellement comportant un ou plusieurs hétéroatomes choisis parmi oxygène, azote, et soufre ;
- a et t, identiques ou différents et indépendamment l'un de l'autre, représentent chacun un entier compris entre 1 et 9, de préférence entre 1 et 3, bornes incluses ;
- b et u, identiques ou différents et indépendamment l'un de l'autre, représentent chacun un entier égal à 1 ou 2 ;
- c et v, identiques ou différents et indépendamment l'un de l'autre, représentent chacun un entier compris entre 1 et 6, bornes incluses ;
- m représente 0, ou un entier compris entre 1 et 20, bornes incluses ;
- n représente un entier compris entre 8 et 20, bornes incluses ;
- p représente un entier compris entre 1 et 10, bornes incluses ;
- étant entendu qu'au moins un des groupements R¹, R² ou R⁴ représente un groupement -(CH₂)ₚ-S-C(XY)-(CH₂)ₙ-C(O)OR³, où R³, X, Y, n et p sont tels que définis ci-dessus.

Par « groupement hydrocarboné linéaire ou ramifié comprenant de 1 à 20 atomes de carbone », on entend des chaînes hydrocarbonées linéaires ou ramifiées, saturées ou insaturées, comportant de 1 à 20 atomes de carbone, de préférence de 1 à 12 atomes de carbone, de préférence encore de 1 à 8 atomes de carbone, par exemple choisies parmi méthyle, éthyle, propyle (n- ou *iso-*propyle), butyle (n-, *iso-* ou *tert*-butyle), pentyle (n-, *iso-* ou *néo*-pentyle), hexyle, hexényle, heptyle, heptényle, octyle, octényle, nonyle, nonényle, décyle, décényle, undécyle, undécényle, dodécyle et dodécényle, de préférence choisies parmi méthyle, éthyle, n-propyle, iso-propyle, n-butyle, *iso*-butyle et *tert*-butyle.

On préfère les composés de formule (1) pour lesquels X et Y représentent chacun l'atome d'hydrogène. Selon un autre mode de réalisation, on préfère les composés de formule (1) pour lesquels X représente le radical méthyle, le radical éthyle ou un radical propyle et Y représente l'atome d'hydrogène. On préfère également les composés de formule (1) pour lesquels R⁴ représente l'atome d'hydrogène.

Dans un mode de réalisation, les composés de formule (1) préférés sont ceux dans lesquels -T- est choisi parmi une liaison simple, -Sᵥ-, -(CH₂)ₜ, -C(CH₃)₂-, -[S(O)ᵤ]ᵥ-, où v représente un entier compris entre 1 et 6, bornes incluses, de préférence 1 à 4, t est un entier compris entre 1 et 9, de préférence entre 1 et 3, bornes incluses, u est de préférence égal à 2.

Selon un autre mode de réalisation, les composés de formule (1) sont ceux pour lesquels m représente 0. On identifiera dans la suite avec la formule (1₀), les composés de formule (1) pour lesquels m représente 0 (zéro). En variante, les composés de formule (1) pour lesquels m est différent de 0 sont identifiés par la formule (1ₘ). Lorsque m est différent de 0, on préfère les composés de formule (1ₘ) pour lesquels m représente un entier compris de préférence entre 1 et 10, bornes incluses, de préférence encore m est égal à 1, 2, 3, 4, 5 ou 6. L'ensemble des composés de formule (1ₘ) avec ceux de formule (1₀) forme l'ensemble des composés de formule (1).

Les composés de formule (1₀) pour lesquels p est strictement supérieur à 1 et inférieur ou égal à 10, avec les composés de formule (1ₘ) pour lesquels p représente un entier compris entre 1 et 10, bornes incluses, forment les composés de formule générale (1"). Ces composés de formule générale (1") sont nouveaux et à ce titre forment un autre objet de la présente invention, comme indiqué plus loin.

Selon encore un autre mode réalisation, on préfère les composés de formule (1) pour lesquels n représente 8, 9, 10, 11 ou 12, de préférence encore 8 ou 9. On préfère également les composés de formule (1) pour lesquels p représente 1, 2, 3, ou 4, de préférence 1 ou 2, de manière tout à fait préférée p est égal à 1.

Un autre mode de réalisation préféré de la présente invention concerne le procédé de préparation des composés de formule (1) comprenant au moins deux, de préférence au moins trois, de préférence encore au moins 4, groupements -(CH₂)ₚ-S-C(XY)-(CH₂)ₙ-C(O)OR³, où R³, X, Y, n et p sont tels que définis précédemment. Dans les composés de formule (1ₘ), il doit être compris que les radicaux R⁷ d'une part et R⁸ d'autre part peuvent être identiques ou différents, et sont de préférence identiques.

On préfère tout particulièrement les composés de formule (1) présentant au moins une, au moins deux, au moins trois, au moins quatre, voire toues les caractéristiques suivantes :
- R¹ est choisi parmi l'atome d'hydrogène, un groupement hydrocarboné linéaire ou ramifié comprenant de 1 à 12 atomes de carbone, et un groupement - (CH₂)ₚ-S-C(XY)-(CH₂)ₙ-C(O)OR³ ;
- R² est choisi parmi un groupement hydrocarboné linéaire ou ramifié comprenant de 1 à 12 atomes de carbone, et un groupement -(CH₂)ₚ-S-C(XY)-(CH₂)ₙ-C(O)OR³ ;
- R³ représente un groupement hydrocarboné linéaire ou ramifié comprenant de 1 à 6 atomes de carbone ;
- R⁴ est choisi parmi l'atome d'hydrogène et un groupement hydrocarboné linéaire ou ramifié comprenant de 1 à 6 atomes de carbone ;
- T est choisi parmi une liaison simple, -Sᵥ-, -(CH₂)ₜ- et -C(CH₃)₂- ;
- X et Y, indépendamment l'un de l'autre, représentent chacun un radical choisi parmi l'atome d'hydrogène et les radicaux alkyle, linéaires ou ramifiés, comportant de 1 à 6 atomes de carbone ;
- t représente un entier compris entre 1 et 3, bornes incluses ;
- v représente un entier compris entre 1 et 6, bornes incluses ;
- m représente 0, ou un entier compris entre 1 et 10, bornes incluses ;
- n représente un entier compris entre 8 et 20, bornes incluses ;
- p représente 1 ou 2, de préférence p est égal à 1 ;
- étant entendu qu'au moins un des groupements R¹ ou R² représente un groupement -(CH₂)ₚ-S-C(XY)-(CH₂)ₙ-C(O)OR³, où R³, X, Y, n et p sont tels que définis ci-dessus.

On préfère tout particulièrement les composés de formule (1) présentant au moins une, de préférence au moins deux, de préférence au moins trois, de préférence au moins quatre, de préférence toutes les caractéristiques suivantes :
- R¹ est choisi parmi l'atome d'hydrogène, et un groupement hydrocarboné linéaire ou ramifié comprenant de 1 à 6 atomes de carbone ;
- R² représente un groupement -(CH₂)ₚ-S-C(XY)-(CH₂)ₙ-C(O)OR³ ;
- R³ représente méthyle ou éthyle ;
- R⁴ représente l'atome d'hydrogène ;
- T est choisi parmi une liaison simple, -Sᵥ-, -(CH₂)ₜ- et -C(CH₃)₂- ;
- X représente un radical choisi parmi l'atome d'hydrogène et les radicaux méthyle, éthyle, propyles et butyles ;
- Y représente l'atome d'hydrogène ;
- t représente un entier compris entre 1 et 3, bornes incluses ;
- v représente un entier compris entre 1 et 6, bornes incluses ;
- m représente 0, ou un entier compris entre 1 et 6, bornes incluses ;
- n représente un entier compris entre 8 et 12, bornes incluses ; et
- p est égal à 1.

Ainsi, la présente invention concerne un procédé de préparation des composés de formule (1) à partir de matières premières d'origine renouvelable, et plus particulièrement à partir d'acides gras d'origine végétale ou animale. Plus particulièrement, la présente invention concerne un procédé de préparation d'un composé de formule (1) tel que défini ci-dessus, ledit procédé comprenant au moins les étapes a) à c) suivantes :
a) réaction d'un mercaptoalcanoate de formule (2) : 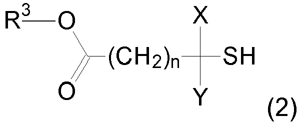 dans laquelle R³, X, Y et n sont tels que définis précédemment, avec un composé aminé de formule (3) : 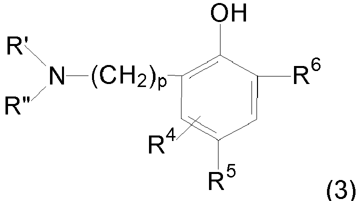 dans laquelle R⁴ et p sont tels que définis précédemment et
   - R' et R", identiques ou différents, sont choisis, indépendamment l'un de l'autre, parmi l'atome d'hydrogène, un radical alkyle linéaire ou ramifié comportant de 1 à 6 atomes de carbone, ou bien forment ensemble et avec l'atome d'azote qui les porte, un hétérocycle,
   - R⁵ représente un groupement hydrocarboné linéaire ou ramifié comprenant de 1 à 20 atomes de carbone ou un groupement -(CH₂)ₚ-NR'R", et
   - R⁶ est choisi parmi l'atome d'hydrogène, un groupement hydrocarboné linéaire ou ramifié comprenant de 1 à 20 atomes de carbone, et un groupement -(CH₂)ₚ-NR'R",
b) éventuellement réaction du composé obtenu lors de l'étape a) avec un composé de formule (4) : 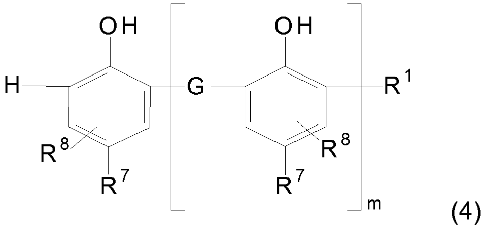 dans laquelle R¹, R⁷, R⁸, m et G sont tels que définis précédemment,
   via une réaction de substitution électrophile aromatique, selon les techniques bien connues de l'homme métier, et,
c) extraction puis purification éventuelle du composé de formule (1).

Parmi les hétérocycles formés par R', R" avec l'atome d'azote qui les porte, on peut citer, de manière non limitative et à titre d'exemples uniquement, les hétérocycles à 4, 5 ou 6 chaînons, saturés ou insaturés, et pouvant contenir un ou plusieurs autres hétéroatomes choisis de préférence parmi oxygène, azote, soufre. De tels hétéroatomes sont bien connus de l'homme du métier, sont par exemple décrits dans la demande US2008/0081929 et sont de préférence la pipéridine la pyrrolidine, et la pipérazine.

La réaction de condensation du composé de formule (2) avec le composé de formule (3) peut être réalisée selon toute méthode connue de l'homme du métier. Cette réaction peut être réalisé en milieu solvant ou sans solvant, éventuellement en présence d'un catalyseur, à une température comprise typiquement, mais pas exclusivement, entre 90°C et 150°C, de préférence à pression atmosphérique, pendant une durée variant entre une heure et quelques heures, par exemple entre 2 heures et 36 heures, selon les substrats considérés, la température et la pression dans le milieu réactionnel.

Les solvants qui peuvent être utilisés dans le procédé de la présente invention sont de tous types connus de l'homme du métier et notamment les solvants organiques, aqueux et hydro-organiques. Des exemples typiques de solvants utilisables dans le procédé de l'invention comprennent l'eau, les alcools (en particulier méthanol, éthanol), les glycols, ainsi que les mélanges de deux ou plusieurs d'entre eux en toutes proportions.

Le composé de formule (4) est soumis à une réaction de substitution électrophile aromatique en présence du composé obtenu lors de l'étape a), éventuellement en présence d'un réactif précurseur du groupe T et éventuellement d'un catalyseur de type acide de Lewis, selon des techniques bien connues de l'homme du métier et par exemple décrites dans « Advanced Organic Chemistry », M. B. Smith & J. March, 5e édition, 2001, Chapitre 11, pp. 675 sqq.

Les composés de formule (4) peuvent être typiquement un phénol (cas des composés de formule (4), où m représente 0) ou une résine phénolique (cas des composés de formule (4), où m est différent de 0). Les composés de formule (4) sont connus et disponibles dans le commerce ou facilement préparés à partir de modes opératoires connus et disponibles dans la littérature scientifique, la littérature brevets, les Chemical Abstracts ou encore l'Internet.

Des exemples non limitatifs de résines phénoliques comprennent, à titre d'illustration de l'invention et de manière non limitative :
- les résines phénol/aldéhyde, de type Novolac, par exemple issues de la réaction de para-alkylphénol avec du paraformaldéhyde en présence d'un acide de Lewis ; dans ce cas, la réaction avec le synthon obtenu lors de l'étape (a) est réalisée de préférence en présence de paraformaldéhyde et d'un acide de Lewis ;
- les résines phénoliques sulfurées, de type Vultac®, pouvant être issues de la réaction de para-alkylphénol avec du chlorure de soufre (et éventuellement du soufre additionnel) ainsi qu'éventuellement un acide de Lewis ;. dans ce cas, la réaction avec le synthon obtenu lors de l'étape (a) est réalisée de préférence en présence de chlorure de soufre ou dichlorure de soufre, et éventuellement d'un acide de Lewis, comme par exemple décrit dans le document WO2005037910 ;
- les résines phénoliques avec des ponts gem-diméthylé issues de la réaction d'oligomérisation de l'*ortho*-isopropényl-para-alkylphénol éventuellement en présence d'un acide de Lewis ; dans ce cas, la réaction avec le synthon obtenu lors de l'étape (a) est réalisée de préférence en présence d'un acide de Lewis.

Les composés de formules (2), (3) et (4) sont connus et disponibles dans le commerce ou sont aisément préparés à partir de modes opératoires connus et disponibles dans les brevets, la littérature scientifique, les Chemical Abstracts, ou sur l'Internet.

De manière tout à fait avantageuse, les composés de formule (2) peuvent être obtenus à partir d'huiles ou de graisses végétales ou animales, et/ou d'acides gras naturels, selon des procédés connus de l'homme de métier.

À titre d'exemples, les composés de formule (2) peuvent être obtenus selon les procédés décrits dans les brevets FR2424907, FR2603889, et dans la demande de brevet US20120232297.

Selon un mode de réalisation particulièrement préféré, les composés de formule (2) sont obtenus à partir d'huiles ou de graisses végétales ou animales, qui sont majoritairement sous la forme de triglycérides. Ces triglycérides sont soumis (étape a1) à une réaction de transestérification catalytique basique ou acide, en présence d'un alcool, de préférence un alcool aliphatique, typiquement du méthanol ou de l'éthanol, pour conduire aux esters d'acides gras correspondant, avec élimination de glycérol.

Les esters d'acides gras comportent généralement et le plus souvent une ou plusieurs double(s) liaison(s), ensuite soumise(s) (étape a) à une réaction de sulfhydratation, afin de conduire au mercapto-ester de formule (2).

En variante, une ou plusieurs réaction(s) de métathèse peu(ven)t être conduite(s) sur les triglycérides et/ou les esters d'acides gras, afin de modifier ou isomériser la ou les double(s) liaison(s) présente(s) sur les chaînes grasses, comme par exemple décrit dans US20120232297.

Les huiles ou graisses végétales ou animales, ci-après « huiles naturelles », qui sont mises en oeuvre à l'étape a1) peuvent être de tous types connus de l'homme du métier, et notamment des triesters de glycérol et d'acides gras (pouvant également contenir des mono- et des di-glycérides) et qui sont trouvés en abondance dans la nature, par exemple dans les plantes oléagineuses et les graisses animales pour ne citer que les sources les plus importantes de triglycérides, en particulier de triglycérides insaturés, c'est-à-dire qu'ils comportent une double liaison carbone-carbone.

Les triglycérides d'acides gras peuvent également contenir une quantité plus ou moins importante d'acides acides gras libres. Lorsque cette quantité est relativement élevée, typiquement supérieure à environ 5% en poids, il peut être avantageux de réaliser un prétraitement des triglycérides consistant en une première estérification desdits acides gras libres en présence de d'un alcool et d'un catalyseur acide, tel que l'acide sulfurique ou l'acide méthane-sulfonique, comme décrit par exemple dans le brevet FR2929621. Les acides gras libres préalablement contenus dans les triglycérides sont ainsi convertis en esters.

Les acides gras libres présents dans les triglycérides de départ peuvent également se trouver en quantités plus faibles, typiquement entre 0,1% en poids et 5% en poids, et dans ces cas, un lavage basique peut être suffisant pour les éliminer sous forme de sels basiques.

L'étape a1) est une étape de trans-estérification de l'huile naturelle (après prétraitement éventuel des acides gras libres, comme indiqué ci-dessus) permettant d'éliminer le glycérol.

Les glycérides insaturés qui peuvent être utilisés proviennent essentiellement d'huiles ou de graisses animales ou végétales, de préférence végétales, parmi lesquelles on peut citer à titre indicatif et non limitatif l'huile de soja, l'huile de tournesol, l'huile de lin, l'huile de colza, l'huile de ricin, l'huile de palme, l'huile de palmiste, l'huile de coprah, l'huile de jatropha, l'huile de coton, l'huile d'arachide, l'huile d'olive, l'huile de vernonia, l'huile de cuphéa, l'huile d'hévéa, l'huile de lunaire, l'huile de carthame, l'huile de camélina, l'huile de *Calophyllum inophyllum,* l'huile de *Pongamia pinnata,* le suif de boeuf, l'huile de cuisson ou de la graisse, mais peuvent aussi être des huiles hydrauliques ou de lubrification.

La réaction de trans-estérification des triglycérides est généralement réalisée en milieu basique, en présence d'un alcool, généralement un monoalcool, comprenant de 1 à 20 atomes de carbone, de préférence de 1 à 12 atomes de carbone, de préférence encore de 1 à 6 atomes de carbone, de manière tout particulièrement préférée de 1 à 4 atomes de carbone, et de manière tout à fait préférée un alcool de faible poids moléculaire, par exemple méthanol ou éthanol, le méthanol étant le plus communément utilisé pour des raisons principalement économiques. La réaction de trans-estérification permet l'obtention d'esters d'acides gras, par exemple d'esters méthyliques lorsque l'alcool utilisé est le méthanol.

Les composés basiques qui peuvent être utilisés pour la réaction de trans-estérification peuvent être de tous types connus de l'homme du métier, et en particulier peuvent choisis parmi les oxydes, hydrures, hydroxydes, carbonates, hydrogénocarbonates, acétates et autres alcoolates de métaux alcalins et alcalino-terreux, les alcoolates provenant d'alcools comportant de préférence de 1 à 5 atomes de carbone. Parmi ces composés basiques, on préfère l'hydroxyde de sodium, l'hydroxyde de potassium, les alcoolates de sodium, les alcoolates de potassium. De manière tout à fait préférée les composés basiques sont choisis parmi l'hydroxyde de sodium, l'hydroxyde de potassium, le méthylate de sodium et le méthylate de potassium, ces deux derniers alcoolates étant tout particulièrement préférés.

On peut également réaliser la réaction de trans-estérification en milieu acide comme indiqué dans la demande internationale WO2011018228. Dans ce cas, on peut par exemple utiliser l'acide méthane-sulfonique en solution aqueuse commercialisé par la société Arkema, par exemple une solution aqueuse d'acide méthane-sulfonique à 70% en poids dans l'eau, ou encore de l'acide méthane-sulfonique anhydre ou AMSA, acronyme pour « anhydrous methane sulphonic acid » en langue anglaise. La trans-estérification en milieu acide présente en outre l'avantage de permettre simultanément l'estérification des acides gras libres éventuellement présents dans les triglycérides d'acides gras.

Après la réaction de trans-estérification, les esters d'acides gras sont présents dans le milieu réactionnel avec le glycérol. Ce milieu réactionnel peut comprendre des quantités plus ou moins importantes d'eau selon les conditions dans lesquelles la réaction de trans-estérification a été conduite. Le glycérol ainsi que l'eau éventuelle ne sont pas solubles dans les esters d'acides gras et sont séparés de ces derniers par décantation ou tout autre moyen permettant la séparation de phases.

Comme indiqué précédemment, les esters insaturés précurseurs des composés de formule (2) peuvent également être obtenus par métathèse croisée à partir d'autres esters insaturés, voire de glycérides (ces derniers seront ensuite soumis à une étape de trans-estérification avec un alcool comme indiqué *supra*), tels que par exemple ceux définis précédemment. Les réactions de métathèse sont bien connues de l'homme du métier et font appel le plus souvent à une réaction intermoléculaire entre deux composés porteurs chacun d'au moins une double liaison, comme décrit par exemple dans les demandes WO2009047444 et US20120232297.

Ces réactions de métathèse sont avantageusement conduites à partir d'esters méthyliques insaturés et par exemple, et de manière non limitative, ceux choisi parmi le palmitoléate de méthyle, l'arachidonate de méthyle, seuls ou mélanges de deux ou plusieurs d'entre eux en toutes proportions. Selon ce mode de réalisation, le 9-octadécén-1,18-dioate de diméthyle peut par exemple être facilement obtenu par métathèse d'oléate de méthyle et/ou de palmitoléate de méthyle.

Les sources d'esters insaturés sont ainsi très nombreuses et variées, et des exemples indicatifs et non limitatifs d'esters méthyliques porteurs d'une insaturation susceptible d'être sulfhydratée comprennent, de manière indicative et non limitative les hexénoates de méthyle, les décénoates de méthyle, les undécénoates de méthyle, les dodécénoates de méthyle, l'oléate de méthyle, le linoléate de méthyle, le myristiléate de méthyle, le palmitoléate de méthyle, le linoléate de méthyle, le linolénate de méthyle, l'arachidonate de méthyle, le ricinoléate de méthyle, le 9-octadécén-1,18-dioate de diméthyle, ainsi que les mélanges de deux ou plusieurs d'entre eux en toutes proportions.

De préférence les esters insaturés sont choisis parmi les décénoates de méthyle et les undécénoates de méthyle, de préférence encore parmi le décén-9-oate de méthyle et le undécén-10-oate de méthyle.

Selon une autre variante, les esters insaturés précurseurs des composés de formule (2) peuvent également être obtenus à partir des acides correspondants, lesquels sont soumis à une réaction d'estérification, en présence d'un alcool, par exemple le méthanol, selon les techniques d'estérification classiques bien connues de l'homme du métier.

Des exemples indicatifs et non limitatifs d'acides précurseurs des esters insaturés comprennent, de manière non limitative les acides hexénoïques, décénoïques, undécénoïques, dodécénoïques, oléique, linoléique, myristique, palmitique, linoléique, linolénique, arachidonique, ricinoléique, les di-acides et triacides qui peuvent être obtenus par métathèse croisée selon les méthodes classiques de synthèse par métathèse, comme indiqué ci-dessus, et par exemple le 9-octadécén-1,18-di-oïque. De préférence, lesdits acides sont choisis parmi les décénoïques et les undécénoïques et les mélanges de deux ou plusieurs d'entre eux en toutes proportions, de préférence encore parmi le décén-9-oïque et le undécén-10-oïque.

Les composés de formule (2) peuvent aisément être préparés à partir des esters insaturés décrits ci-dessus par réaction de sulfhydratation (étape a2) selon les techniques connues de l'homme du métier. Par « réaction de sulfhydratation », on entend l'introduction d'un groupement -SH sur une insaturation comme illustrée dans le schéma ci-dessous :

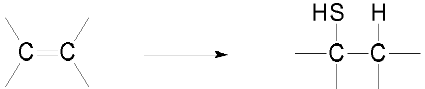

La double liaison carbone-carbone présente dans l'ester insaturé peut ainsi être sulfhydratée en une ou deux étapes, selon une réaction classique d'addition radicalaire par action du sulfure d'hydrogène (comme décrit par exemple dans FR2424907) ou d'un précurseur de sulfure d'hydrogène, par exemple l'acide thio-acétique (comme décrit par exemple dans US4701492), un mercaptan tertiaire, par exemple le *tert*-butylmercaptan (comme décrit par exemple dans FR2603889), ou par une addition catalytique d'hydrogène sulfuré (comme décrit par exemple dans US4102931).

Ainsi, l'agent de sulfhydratation mis en oeuvre pour la sulfhydratation de l'ester insaturé en composé de formule (2) peut être de tout type connu de l'homme du métier et par exemple choisi parmi le sulfure d'hydrogène, l'acide thio-acétique (ATA), et autres composés connus de l'homme du métier et habituellement utilisés dans les réactions de sulfhydratation de composés organiques.

Cette réaction de sulfhydratation est avantageusement conduite en présence de catalyseur acide homogène ou hétérogène et/ou sous irradiation de lumière ultra-violette (U.V.), soit par photolyse directe à des longueurs d'ondes comprises entre 180 nm et 300 nm, soit en présence de photo-initiateurs. Selon un mode de réalisation préféré la réaction de sulfhydratation est conduite sans catalyseur, et sous irradiation U.V.

Cette réaction de sulfhydratation peut être conduite en présence ou en absence de solvant, de préférence en présence d'un ou plusieurs solvants qui peuvent être avantageusement choisis pour leur transparence à la lumière U.V. selon la longueur d'onde utilisée et la facilité de leur séparation du milieu réactionnel. De tels solvants peuvent par exemple être choisis parmi les alcanes légers (1 à 6 atomes de carbone), les éthers d'éthylène-glycol, les hydrocarbures aromatiques, les hydrocarbures aliphatiques, et autres, ainsi que les mélanges de deux ou plusieurs d'entre eux en toutes proportions.

En variante, la réaction de sulfhydratation peut être réalisée en présence d'un ou plusieurs, de préférence un, composé(s) capable(s) de former des radicaux libres. De tels composés sont connus de l'homme du métier et peuvent être choisis par exemple parmi les peroxydes, et de manière indicative et non limitative, parmi le peroxyde d'hydrogène, le peroxyde de sodium ou de potassium, les hydroperoxydes de *tert*-alkyle (par exemple de *tert*-butyle), les peroxydes de *tert*-alkyle, les peresters de *tert*-alkyle, l'hydroperoxyde de cumène, l'azo-bis-*iso*-butyronitrile, et autres, et les mélanges de deux ou plusieurs d'entre eux en toutes proportions.

Lorsque la réaction de sulfhydratation décrite ci-dessus est effectuée par action de l'acide thio-acétique en présence d'un initiateur de radicaux libres et/ou par irradiation à la lumière U.V., comme décrit précédemment, cette réaction est suivie d'une réaction de méthanolyse en milieu acide, permettant de libérer le mercaptan de formule (2) souhaité ainsi que de l'acétate de méthyle éliminé (par exemple et de façon non limitative) en continu du milieu par distillation azéotropique. Cette réaction de méthanolyse est bien connue et peut être réalisée selon toutes techniques classiques.

À l'issue de l'étape de sulfhydratation (étape a2), les mercapto-esters de formule (2) peuvent être obtenus sous forme de mélanges d'isomères (mercaptans primaires, secondaires et/ou tertiaires) qui peuvent être ensuite séparés et éventuellement purifiés selon des techniques classiques de séparation et/ou de purification, par exemple par distillation, sous pression atmosphérique ou sous pression réduite selon la nature du mercaptan d'intérêt à récupérer.

En variante, et selon encore un autre mode de réalisation, les esters insaturés précurseurs des composés de formule (2) peuvent être obtenus à partir d'esters gras insaturés, voire de glycérides, tels que par exemple ceux définis précédemment, ces derniers étant alors préalablement soumis à une étape de trans-estérification avec un alcool comme indiqué *supra.* Ces esters gras insaturés peuvent ensuite être traités par pyrolyse selon des techniques bien connues de l'homme du métier. Ce traitement par pyrolyse est particulièrement bien adapté au traitement du ricinoléate de méthyle, pour lequel la pyrolyse permet d'obtenir de manière sélective le undécén-10-oate de méthyle.

Les esters insaturés résultants d'un ou plusieurs de ces traitements (transestérification, métathèse, pyrolyse et autres), et après séparation des produits de réaction (notamment diesters insaturés, monoesters insaturés, alcènes, et autres) sont ensuite soumis à une réaction de sulfhydratation comme décrit précédemment pour obtenir les composés de formule (2).

Des exemples de composés de formule (2) particulièrement préférés pour la synthèse des composés de formule (1) selon la présente invention sont, de manière non limitative le mercaptodécanoate de méthyle, le mercapto-undécanoate de méthyle.

Le procédé de préparation des composés de formule (1) selon l'invention comprend également la réaction d'au moins un composé de formule (2) décrit ci-dessus, avec un dérivé phénolique de formule (3), comme défini plus haut.

Les dérivés phénoliques de formule (3) sont des dérivés phénoliques aminés connus et aisément accessibles dans le commerce ou encore facilement préparés à partir de modes opératoires connus dans les brevets, la littérature scientifique, les Chemical Abstracts ou l'Internet. Ces composés peuvent également être des intermédiaires de synthèse non isolés comme décrits dans la littérature (US2008081929).

Chaque groupement aminé du composé de formule (3) réagit avec un mercapto-ester de formule (2) dans les conditions connues et décrites dans la littérature, par exemple dans les documents US2008081929 et US 4857572.

Des exemples de composés de formule (3) comprennent, à titre d'exemples non limitatifs, le 2-(N,N-diméthylamino-éthyl)phénol (n° CAS 94-54-2), le 2,4-bis[(N,N-diméthylamino)méthyl]-6-méthylphénol (n° CAS 5424-54-4), le 2,4,6-tris(N,N-diméthylaminométhyl)phénol (n° CAS 90-72-2).

Les composés obtenus après réaction d'un composé de formule (2) avec un composé de formule (3) correspondent aux composés de formule (1_{A}) dans laquelle T représente une liaison et A représente R¹.

Les composés de formule (1), autres que les composés de formule (1_{A}), c'est-à-dire les composés pour lesquels T ne représente pas une liaison et/ou A représente le radical (A^{m}) peuvent aisément être obtenus par réaction d'un réactif porteur du groupe T et/ou d'un composé de formule (4) définie précédemment, comme indiqué *supra,* selon des méthodes bien connues de l'homme du métier.

À titre d'exemple et de manière non limitative :
- les composés de formule (1) pour lesquels T représente S peuvent être obtenus à partir d'un composé de formule (1_{A}) telle que précédemment définie avec R¹ représentant l'atome d'hydrogène, et d'un composé de formule (4), en présence de dichlorure de soufre (SCl₂) ou de chlorure de soufre (S₂Cl₂), en présence d'un solvant, à une température comprise entre -10°C et 40°C, pendant une durée généralement comprise entre 30 minutes et 3 heures ;
- les composés de formule (1) pour lesquels T représente S_{c}, où c représente 2, 3, 4, 5 ou 6, peuvent être obtenus à partir d'un composé de formule (1_{A}) telle que définie précédemment avec R¹ représentant l'atome d'hydrogène, et d'un composé de formule (4), en présence de chlorure de soufre (S₂Cl₂), éventuellement de soufre (S₈) et éventuellement d'un solvant organique, à une température comprise entre 50°C et 160°C, pendant une durée généralement comprise entre 30 minutes et 3 heures ;
- les composés de formule (1) pour lesquels T représente -(CH₂)- peuvent être obtenus à partir d'un composé de formule (1_{A}), telle que précédemment définie avec R¹ représentant l'atome d'hydrogène, et d'un composé de formule (4), en présence de formaldéhyde (CH₂O), à une température généralement comprise entre 60°C et 150°C ;
- les composés de formule (1) pour lesquels T représente -C(CH₃)₂-, peuvent être obtenus à partir d'un composé de formule (1_{A}) pour lequel R¹ représente un groupement iso-propényle et d'un composé de formule (4), en présence d'un acide de Lewis, tel que le triéthylaluminium, d'un solvant et dans une gamme de température allant de 60°C à 150°C, pour une durée pouvant aller de 30 minutes à 6 heures, et
- les composés de formule (1) pour lesquels T représente -[S(O)_{b}]_{c}-, peuvent être obtenus à partir d'un composé de formule (1_{A}) pour lequel T représente S_{c}, où c représente 2, 3, 4, 5 ou 6, en présence d'un agent oxydant (tel que le peroxyde d'hydrogène) et d'un solvant organique, à température comprise entre 0°C et 40°C pendant une durée comprise entre 1 heure et 48 heures.

Le procédé selon la présente invention permettant de préparer les composés de formule (1), telle que précédemment définie, à partir d'un triglycéride, de préférence d'origine naturelle, comprend ainsi au moins les étapes suivantes :
a0) fourniture d'au moins un triglycéride ;
a1) transestérification dudit au moins un triglycéride, en présence d'un alcool et élimination du glycérol formé, pour obtenir un ester insaturé ;
a1') traitement éventuel par métathèse ou pyrolyse dudit ester insaturé ;
a2) sulfhydratation de l'ester insaturé de l'étape a1) ou a1') pour obtenir le mercapto-ester de formule (2), telle que définie précédemment ;
   a) réaction du mercapto-ester de formule (2) telle que définie précédemment avec un composé phénolique aminé de formule (3) telle que définie précédemment, pour obtenir les composés de formule (1_{A}) telle que définie précédemment ;
   b) éventuellement réaction du composé obtenu à l'étape a) avec un composé de formule (4), éventuellement en présence d'un réactif porteur du groupe T ;
   c) extraction puis purification éventuelle des composés obtenus à l'étape b).

Comme indiqué précédemment, les composés de formule (1₀) pour lesquels p est strictement supérieur à 1 et inférieur ou égal à 10, avec les composés de formule (1ₘ) pour lesquels p représente un entier compris entre 1 et 10, bornes incluses, sont nouveaux et à ce titre forment un autre objet de la présente invention.

Ainsi, un autre objet de la présente invention concerne les composés de formule (1") : 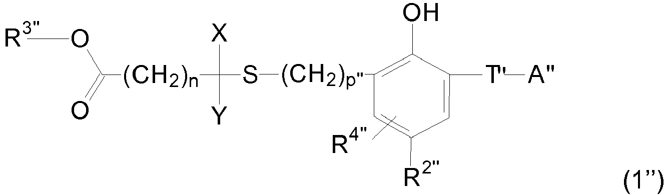 dans laquelle :
- A" représente un radical R¹" ou un radical de formule A^{m}" : 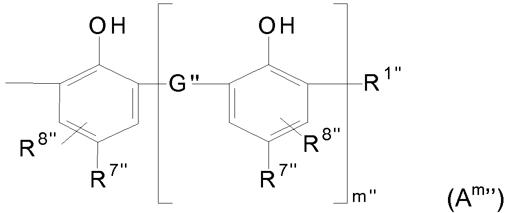
- R¹" est choisi parmi l'atome d'hydrogène, un groupement hydrocarboné linéaire ou ramifié comprenant de 1 à 20 atomes de carbone, et un groupement -(CH₂)_{p"}-S-C(X"Y")-(CH₂)_{n"}-C(O)OR³" ;
- R²" est choisi parmi un groupement hydrocarboné linéaire ou ramifié comprenant de 1 à 20 atomes de carbone, et un groupement -(CH₂)_{p"}-S-C(X"Y")-(CH₂)_{n"}-C(O)OR³" ;
- R³" représente un groupement hydrocarboné linéaire ou ramifié comprenant de 1 à 20 atomes de carbone ;
- R⁴", R⁷" et R⁸", identiques ou différents, sont choisis, indépendamment l'un de l'autre, parmi l'atome d'hydrogène, un groupement hydrocarboné linéaire ou ramifié comprenant de 1 à 20 atomes de carbone, et un groupement -(CH₂)_{p"}-S-C(X"Y")-(CH₂)_{n"}-C(O)OR³" ;
- G" est choisi parmi -S_{c"}-, -(CH₂)_{a"}-, -C(CH₃)₂-, -[S(O)_{b"}]_{c"}- et -W"-, où W" est un groupement aromatique, éventuellement substitué par un ou plusieurs groupes alkyle ;
- T" est choisi parmi une liaison simple, -S_{v"}-, -(CH₂)_{t"}-, -C(CH₃)₂-, et -[S(O)_{u"}]_{v"}- ;
- X" et Y", indépendamment l'un de l'autre, représentent chacun un radical choisi parmi l'atome d'hydrogène et les groupements hydrocarbonés, linéaires ou ramifiés, comportant de 1 à 20 atomes de carbone, et éventuellement comportant un ou plusieurs hétéroatomes choisis parmi oxygène, azote, et soufre ;
- a" et t", identiques ou différents et indépendamment l'un de l'autre, représentent chacun un entier compris entre 1 et 9, de préférence entre 1 et 3, bornes incluses ;
- b" et u", identiques ou différents et indépendamment l'un de l'autre, représentent chacun un entier égal à 1 ou 2 ;
- c" et v", identiques ou différents et indépendamment l'un de l'autre, représentent chacun un entier compris entre 1 et 6, bornes incluses ;
- m" représente 0, ou un entier compris entre 1 et 20, bornes incluses ;
- n" représente un entier compris entre 8 et 20, bornes incluses ;
- p" représente un entier compris entre 1 et 10, bornes incluses ;
- étant entendu qu'au moins un des groupements R¹", R²" ou R⁴" représente un groupement -(CH₂)_{p"}-S-C(X"Y")-(CH₂)ₙ-C(O)OR³", où R³", X", Y", n" et p" sont tels que définis ci-dessus, et
- étant entendu que lorsque p" = 1, alors A" représente un radical de formule A^{m}".

L'ensemble des composés de formule (1") est compris dans l'ensemble des composés de formule (1). Ainsi, les composés de formule (1") peuvent être préparés selon le mode opératoire décrit pour l'obtention des composés de formule (1). De même, et sauf indication contraire, les définitions et préférences données pour les divers substituants des composés de formule (1) s'appliquent également aux substituants des composés de formule (1").

En particulier, on préfère les composés de formule (1") pour lesquels X" et Y" représentent chacun l'atome d'hydrogène. On préfère également les composés de formule (1") pour lesquels X" représente le radical méthyle, le radical éthyle ou un radical propyle et Y" représente l'atome d'hydrogène. On préfère en outre les composés de formule (1") pour lesquels R⁴" représente l'atome d'hydrogène.

Dans un mode de réalisation, les composés de formule (1") préférés sont ceux dans lesquels -T"- est choisi parmi une liaison simple, -S_{v"}-, -(CH₂)_{t"}-, -C(CH₃)₂-, -[S(O)_{u"}]_{t"}-, où v" représente un entier compris entre 1 et 6, bornes incluses, de préférence 1 à 4, t" est de préférence un entier compris entre 1 et 9 et de préférence entre 1 et 3, bornes incluses, u" est de préférence égal à 2.

On préfère également les composés de formule (1") pour lesquels m" représente un entier égal à 1, 2, 3, 4, 5 ou 6 et p" représente un entier égal à 1, 2, 3 ou 4.

Selon encore un autre mode réalisation, on préfère les composés de formule (1") pour lesquels n" représente 8, 9, 10, 11 ou 12, de préférence encore 8 ou 9. On préfère également les composés de formule (1") pour lesquels m" est égal à 1, 2, 3 ou 4, et p" représente 2, 3, ou 4, de préférence 2.

Un autre mode de réalisation préféré de la présente invention regroupe les composés de formule (1") comprenant 2, 3 ou 4, de préférence 2 ou 3, de préférence 2 groupements -(CH₂)_{p"}-S-C(X"Y")-(CH₂)_{n"}-C(O)OR³", où R³", X", Y", n" et p" sont tels que définis précédemment. Dans les composés de formule (1"), il doit être compris que les radicaux R²" peuvent être identiques ou différents, et sont de préférence identiques.

On préfère tout particulièrement les composés de formule (1") présentant au moins une, au moins deux, au moins trois, au moins quatre, voire toutes les caractéristiques suivantes :
- R¹" est choisi parmi l'atome d'hydrogène, un groupement hydrocarboné linéaire ou ramifié comprenant de 1 à 12 atomes de carbone, et un groupement -(CH₂)_{p"}-S-C(X"Y")-(CH₂)_{n"}-C(O)OR³" ;
- R²" est choisi parmi un groupement hydrocarboné linéaire ou ramifié comprenant de 1 à 12 atomes de carbone, et un groupement -(CH₂)_{p"}-S-C(X"Y")-(CH₂)_{n"}-C(O)OR³" ;
- R³" représente un groupement hydrocarboné linéaire ou ramifié comprenant de 1 à 6 atomes de carbone ;
- R⁴" est choisi parmi l'atome d'hydrogène et un groupement hydrocarboné linéaire ou ramifié comprenant de 1 à 6 atomes de carbone ;
- T" est choisi parmi une liaison simple, -S_{v"}-, -(CH₂)_{t"}- et -C(CH₃)₂- ;
- X" et Y", indépendamment l'un de l'autre, représentent chacun un radical choisi parmi l'atome d'hydrogène et les radicaux alkyle, linéaires ou ramifiés, comportant de 1 à 6 atomes de carbone ;
- t" représente un entier compris entre 1 et 3, bornes incluses ;
- v" représente un entier compris entre 1 et 6, bornes incluses ;
- m" représente 0, ou un entier compris entre 1 et 10, bornes incluses ;
- n" représente un entier compris entre 8 et 20, bornes incluses ;
- p" représente 2 ;
- étant entendu qu'au moins un des groupements R¹" ou R²" représente un groupement -(CH₂)_{p"}-S-C(X"Y)"-(CH₂)_{n"}-C(O)OR³", où R³", X", Y", n" et p" sont tels que définis ci-dessus,
et de préférence encore ceux présentant au moins une, au moins deux, au moins trois, au moins quatre, voire toutes les caractéristiques suivantes :
- R¹" est choisi parmi l'atome d'hydrogène, et un groupement hydrocarboné linéaire ou ramifié comprenant de 1 à 6 atomes de carbone ;
- R²" représente un groupement -(CH₂)_{p"}-S-C(X"Y")-(CH₂)_{n"}-C(O)OR³" ;
- R³" représente méthyle ou éthyle ;
- R⁴" représente l'atome d'hydrogène ;
- T" est choisi parmi une liaison simple, -Sᵥ-, -(CH₂)_{t"}- et -C(CH₃)₂- ;
- X" représente un radical choisi parmi l'atome d'hydrogène et les radicaux méthyle, éthyle, propyles et butyles ;
- Y" représente l'atome d'hydrogène ;
- t" représente un entier compris entre 1 et 3, bornes incluses ;
- v" représente un entier compris entre 1 et 6, bornes incluses ;
- m" représente 0, ou un entier compris entre 1 et 6, bornes incluses ;
- n" représente un entier compris entre 8 et 12, bornes incluses ; et
- p" est égal à 1.

Grâce à la présente invention, il est maintenant possible de proposer de nouveaux composés phénoliques soufrés qui sont moins toxiques, plus respectueux de l'environnement, et ne présentant que peu ou pas d'odeurs, en particulier d'odeurs désagréables, telles que celles que l'on peut ressentir avec certains composés phénoliques soufrés connus et qui comportent des traces de produits de départ nauséabonds qui n'ont pas réagi, tels les mercaptans, en particulier certains n-alkylmercaptans.

Les composés phénoliques selon la présente invention présentent de bonnes propriétés en tant qu'agents anti-oxydants, et particulièrement des propriétés améliorées par rapport aux agents anti-oxydants de la famille des composés phénoliques connus de l'art antérieur, ce qui permet leur utilisation comme anti-oxydants, stabilisants U.V., stabilisants thermiques, et autres.

Ainsi, et selon encore un autre aspect, la présente invention concerne l'utilisation d'au moins un composé phénolique soufré de formule (1") telle que définie ci-dessus, comme anti-oxydant, stabilisant U.V., stabilisant thermique, dans de nombreuses applications, et particulièrement dans la préparation de matières plastiques, fibres synthétiques, élastomères, adhésifs, additifs, lubrifiants, etc....

Les exemples suivants illustrent l'invention sans la limiter.

### Exemple 1 : Préparation du 2-(10-méthyl(éthylthio)décanoate)phénol à partir d'huile de soja

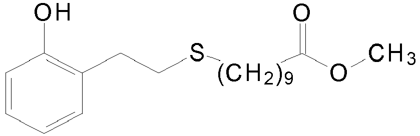

De l'huile de soja est traitée avec de l'alumine afin de réduire sa teneur en peroxydes à moins de 10 milliéquivalents par kg d'huile de soja. L'huile de soja est ensuite dégazée par barbotage d'azote pendant 30 minutes. L'huile de soja traitée est ensuite stockée dans un récipient sous atmosphère d'azote jusqu'à utilisation.

Dans un autoclave de 500 mL en acier inoxydable, équipé d'un dispositif d'agitation magnétique, d'un dispositif de chauffe, d'une vanne d'introduction de gaz et d'une soupape, on introduit 250 mL d'huile de soja déperoxydée et dégazée, comme précédemment).

Un tube de verre scellé contenant 35 mg de chlorure de (tricyclohexyl-phosphine)(benzylidène)ruthénium dissous dans 5 mL de toluène est inséré dans l'autoclave. L'autoclave est fermé, puis mis sous atmosphère inerte d'azote par 3 cycles de purge sous vide / mise sous légère pression d'azote (550 kPa). Le système est ensuite purgé sous vide une dernière fois avant d'introduire l'éthylène dans le milieu, en maintenant une pression de 2,7 MPa, et qui sera maintenue pendant la durée de l'essai.

L'agitation est lancée pour briser le tube de verre contenant le catalyseur puis la température du milieu montée à 30°C et maintenue pendant 10 heures.

En fin de test, l'autoclave est refroidi, ramené à pression atmosphérique et le contenu purifié par passage sur alumine pour éliminer le chlorure de (tricyclohexylphosphine)(benzylidène)ruthénium. Le milieu réactionnel est ensuite purifié par distillation pour séparer les sous-produits du tris-(9-décénoate) de glycérol. Le produit attendu est obtenu avec un rendement supérieur à 70%.

Une étape de méthanolyse des produits est réalisée sur le tris-(9-décénoate) de glycérol afin de récupérer d'une part le glycérol et le 9-décénoate de méthyle d'autre part.

Le 9-décénoate de méthyle ainsi obtenu (156 g) est introduit dans un réacteur photochimique comprenant une boucle réactionnelle, avec 100 g de pentane et 60 équivalents molaires de sulfure d'hydrogène liquéfié (1806 g condensés à 20°C sous une pression de 17,5 bars soit 1,75 MPa). Le mélange est recirculé à raison de 60 L/h dans la boucle réactionnelle au sein de laquelle il est soumis à des radiations U.V. (longueur d'onde: 254 nm, puissance: 12 Watt) pendant 3 heures à une température de 38°C, et une pression de 23 bars (2,3 MPa).

L'excès de sulfure d'hydrogène est ensuite purgé vers un oxydateur thermique par décompression du milieu, puis par un strippage à l'azote. Le mélange est ensuite distillé afin d'éliminer le solvant et les sulfures formés, à une température de 130°C, sous une pression de 5 mbar (500 Pa). Le mercapto-9-décanoate de méthyle est obtenu avec une pureté supérieure à 98,5 %.

Dans un réacteur double enveloppe équipé d'un système d'agitation et d'un réfrigérant, on mélange 115,7 g (0,7 moles) de 2 (diméthylaminoéthyl)phénol et 152,7 g de 10-mercaptodécanoate de méthyle (0,7 moles) pendant une durée de 36 heures à 150°C. La diméthylamine est retirée du milieu en continu par léger stripping à l'azote.

Le brut réactionnel est retiré du milieu, lavé à l'eau et la phase organique distillée sous pression réduite pour donner le 2-(10-méthyl(éthylthio)-décanoate)phénol attendu.

## Revendications

1. Procédé de préparation, à partir de matières premières d'origine renouvelable, de composés phénoliques soufrés répondant à la formule (1) suivante : 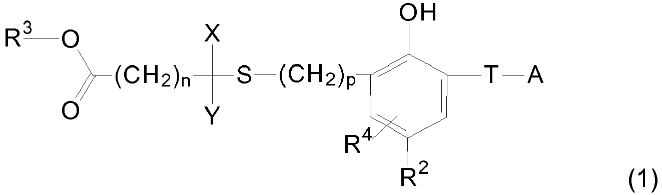 dans laquelle :
• A représente un radical R¹ ou un radical de formule A^{m} : 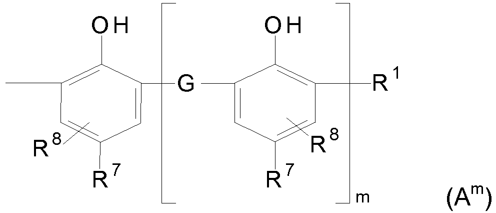
• R¹ est choisi parmi l'atome d'hydrogène, un groupement hydrocarboné linéaire ou ramifié comprenant de 1 à 20 atomes de carbone, et un groupement - (CH₂)ₚ-S-C(XY)-(CH₂)ₙ-C(O)OR³ ;
• R² est choisi parmi un groupement hydrocarboné linéaire ou ramifié comprenant de 1 à 20 atomes de carbone, et un groupement -(CH₂)ₚ-S-C(XY)-(CH₂)ₙ-C(O)OR³ ;
• R³ représente un groupement hydrocarboné linéaire ou ramifié comprenant de 1 à 20 atomes de carbone ;
• R⁴, R⁷ et R⁸, identiques ou différents, sont choisis, indépendamment l'un de l'autre, parmi l'atome d'hydrogène, un groupement hydrocarboné linéaire ou ramifié comprenant de 1 à 20 atomes de carbone, et un groupement -(CH₂)ₚ-S-C(XY)-(CH₂)ₙ-C(O)OR³ ;
• G est choisi parmi -S_{c}-, -(CH₂)ₐ-, -C(CH₃)₂-, -[S(O)_{b}]_{c}- et -W-, où W est un groupement aromatique, éventuellement substitué par un ou plusieurs groupes alkyle ;
• T est choisi parmi une liaison simple, -Sᵥ-, -(CH₂)ₜ-, -C(CH₃)₂-, et -[S(O)ᵤ]ᵥ- ;
• X et Y, indépendamment l'un de l'autre, représentent chacun un radical choisi parmi l'atome d'hydrogène et les groupements hydrocarbonés, linéaires ou ramifiés, comportant de 1 à 20 atomes de carbone, et éventuellement comportant un ou plusieurs hétéroatomes choisis parmi oxygène, azote, et soufre ;
• a et t, identiques ou différents et indépendamment l'un de l'autre, représentent chacun un entier compris entre 1 et 9, de préférence entre 1 et 3, bornes incluses ;
• b et u, identiques ou différents et indépendamment l'un de l'autre, représentent chacun un entier égal à 1 ou 2 ;
• c et v, identiques ou différents et indépendamment l'un de l'autre, représentent chacun un entier compris entre 1 et 6, bornes incluses ;
• m représente 0, ou un entier compris entre 1 et 20, bornes incluses ;
• n représente un entier compris entre 8 et 20, bornes incluses ;
• p représente un entier compris entre 1 et 10, bornes incluses ;
• étant entendu qu'au moins un des groupements R¹, R² ou R⁴ représente un groupement -(CH₂)ₚ-S-C(XY)-(CH₂)ₙ-C(O)OR³, où R³, X, Y, n et p sont tels que définis ci-dessus,
ledit procédé comprenant au moins les étapes a) à c) suivantes :
a) réaction d'un mercaptoalcanoate de formule (2) : 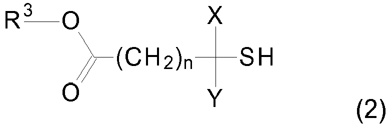 dans laquelle R³, X, Y et n sont tels que définis précédemment, avec un composé aminé de formule (3) : 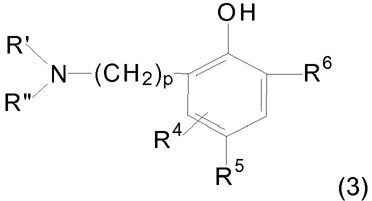 dans laquelle R⁴ et p sont tels que définis précédemment et
• R' et R", identiques ou différents, sont choisis, indépendamment l'un de l'autre, parmi l'atome d'hydrogène, un radical alkyle linéaire ou ramifié comportant de 1 à 6 atomes de carbone, ou bien forment ensemble et avec l'atome d'azote qui les porte, un hétérocycle,
• R⁵ représente un groupement hydrocarboné linéaire ou ramifié comprenant de 1 à 20 atomes de carbone ou un groupement -(CH₂)ₚ-NR'R", et
• R⁶ est choisi parmi l'atome d'hydrogène, un groupement hydrocarboné linéaire ou ramifié comprenant de 1 à 20 atomes de carbone, et un groupement -(CH₂)ₚ-NR'R",
b) éventuellement réaction du composé obtenu lors de l'étape a) avec un composé de formule (4) : 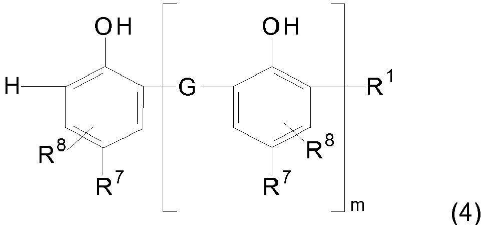 dans laquelle R¹, R⁷, R⁸, m et G sont tels que définis précédemment,
via une réaction de substitution électrophile aromatique, et,
c) extraction puis purification éventuelle du composé de formule (1).

2. Procédé selon la revendication 1, dans lequel les radicaux X et Y du composé de formule (1) représentent chacun l'atome d'hydrogène.

3. Procédé selon la revendication 1 ou la revendication 2, dans lequel le radical R⁴ du composé de formule (1) représente l'atome d'hydrogène.

4. Procédé selon l'une quelconque des revendications précédentes, dans lequel le composé de formule (1) présente au moins une, de préférence au moins deux, de préférence au moins trois, de préférence au moins quatre, de préférence toutes les caractéristiques suivantes :
• R¹ est choisi parmi l'atome d'hydrogène, et un groupement hydrocarboné linéaire ou ramifié comprenant de 1 à 6 atomes de carbone ;
• R² représente un groupement -(CH₂)ₚ-S-C(XY)-(CH₂)ₙ-C(O)OR³ ;
• R³ représente méthyle ou éthyle ;
• R⁴ représente l'atome d'hydrogène ;
• T est choisi parmi une liaison simple, -Sᵥ-, -(CH₂)ₜ- et -C(CH₃)₂- ;
• X représente un radical choisi parmi l'atome d'hydrogène et les radicaux méthyle, éthyle, propyles et butyles ;
• Y représente l'atome d'hydrogène ;
• t représente un entier compris entre 1 et 3, bornes incluses ;
• v représente un entier compris entre 1 et 6, bornes incluses ;
• m représente 0, ou un entier compris entre 1 et 6, bornes incluses ;
• n représente un entier compris entre 8 et 12, bornes incluses ; et
• p est égal à 1.

5. Procédé selon l'une quelconque des revendications précédentes, dans lequel le composé de formule (4) est choisi parmi les résines phénol/aldéhyde, les résines phénoliques sulfurées et les résines phénoliques avec des ponts gem-diméthylé issues de la réaction d'oligomérisation de l'*ortho*-isopropényl-para-alkylphénol.

6. Procédé selon l'une quelconque des revendications précédentes, comprenant au moins les étapes suivantes :
a0) fourniture d'au moins un triglycéride ;
a1) transestérification dudit au moins un triglycéride, en présence d'un alcool et
élimination du glycérol formé, pour obtenir un ester insaturé ;
a1') traitement éventuel par métathèse ou pyrolyse dudit ester insaturé ;
a2) sulfhydratation de l'ester insaturé de l'étape a1) ou a1')pour obtenir le mercapto-ester de formule (2), telle que définie précédemment ;
a) réaction du mercapto-ester de formule (2) telle que définie à la revendication 1 avec un composé phénolique aminé de formule (3) telle que définie à la revendication 1 ;
b) éventuellement réaction du composé obtenu à l'étape a) avec un composé de formule (4) telle que définie à la revendication 1, éventuellement en présence d'un réactif porteur du groupe T tel que défini à la revendication 1 ;
c) extraction puis purification éventuelle des composés obtenus à l'étape b).

7. Procédé selon l'une quelconque des revendications précédentes, dans lequel la matière première d'origine renouvelable est un triglycéride provenant d'huiles ou de graisses animales ou végétales, de préférence végétales, choisies parmi l'huile de soja, l'huile de tournesol, l'huile de lin, l'huile de colza, l'huile de ricin, l'huile de palme, l'huile de palmiste, l'huile de coprah, l'huile de jatropha, l'huile de coton, l'huile d'arachide, l'huile d'olive, l'huile de vernonia, l'huile de cuphéa, l'huile d'hévéa, l'huile de lunaire, l'huile de carthame, l'huile de camélina, l'huile de *Calophyllum inophyllum,* l'huile de *Pongamia pinnata,* le suif de boeuf, l'huile de cuisson ou de la graisse, mais peuvent aussi être des huiles hydrauliques ou de lubrification.

8. Composé de formule (1") : 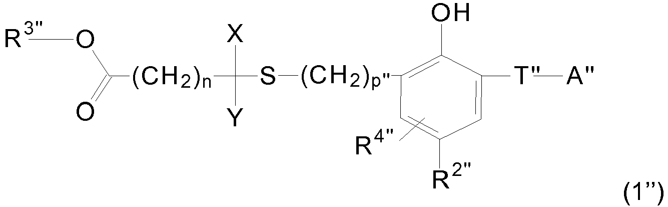 dans laquelle :
• A" représente un radical R¹" ou un radical de formule A^{m}" : 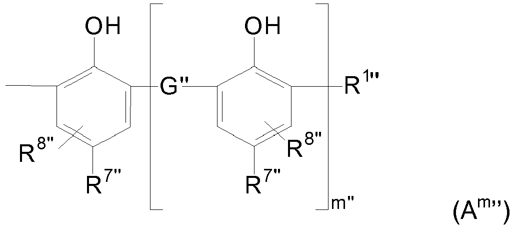
• R¹" est choisi parmi l'atome d'hydrogène, un groupement hydrocarboné linéaire ou ramifié comprenant de 1 à 20 atomes de carbone, et un groupement - (CH₂)_{p"}-S-C(X"Y")-(CH₂)_{n"}-C(O)OR³" ;
• R²" est choisi parmi un groupement hydrocarboné linéaire ou ramifié comprenant de 1 à 20 atomes de carbone, et un groupement -(CH₂)_{p"}-S-C(X"Y")-(CH₂)_{n"}-C(O)OR³" ;
• R³" représente un groupement hydrocarboné linéaire ou ramifié comprenant de 1 à 20 atomes de carbone ;
• R⁴", R⁷" et R⁸", identiques ou différents, sont choisis, indépendamment l'un de l'autre, parmi l'atome d'hydrogène, un groupement hydrocarboné linéaire ou ramifié comprenant de 1 à 20 atomes de carbone, et un groupement -(CH₂)_{p"}-S-C(X"Y")-(CH₂)_{n"}-C(O)OR³" ;
• G" est choisi parmi -S_{c"}-, -(CH₂)_{a"}-, -C(CH₃)₂-, -[S(O)_{b"}]_{c"}- et -W"-, où W" est un groupement aromatique, éventuellement substitué par un ou plusieurs groupes alkyle ;
• T" est choisi parmi une liaison simple, -S_{v"}-, -(CH₂)_{t"}-, -C(CH₃)₂-, et -[S(O)_{u"}]_{v"}- ;
• X" et Y", indépendamment l'un de l'autre, représentent chacun un radical choisi parmi l'atome d'hydrogène et les groupements hydrocarbonés, linéaires ou ramifiés, comportant de 1 à 20 atomes de carbone, et éventuellement comportant un ou plusieurs hétéroatomes choisis parmi oxygène, azote, et soufre ;
• a" et t", identiques ou différents et indépendamment l'un de l'autre, représentent chacun un entier compris entre 1 et 9, de préférence entre 1 et 3, bornes incluses ;
• b" et u", identiques ou différents et indépendamment l'un de l'autre, représentent chacun un entier égal à 1 ou 2 ;
• c" et v", identiques ou différents et indépendamment l'un de l'autre, représentent chacun un entier compris entre 1 et 6, bornes incluses ;
• m" représente 0, ou un entier compris entre 1 et 20, bornes incluses ;
• n" représente un entier compris entre 8 et 20, bornes incluses ;
• p" représente un entier compris entre 1 et 10, bornes incluses ;
• étant entendu qu'au moins un des groupements R¹", R²" ou R⁴" représente un groupement -(CH₂)_{p"}-S-C(X"Y")-(CH₂)ₙ-C(O)OR³", où R³", X", Y", n" et p" sont tels que définis ci-dessus, et
• étant entendu que lorsque p" = 1, alors A" représente un radical de formule A^{m}".

9. Composé selon la revendication 8, dans lequel les radicaux X" et Y" représentent chacun l'atome d'hydrogène.

10. Composé selon la revendication 8 ou la revendication 9, dans lequel le radical R⁴" représente l'atome d'hydrogène.

11. Composé selon l'une quelconque des revendications 8 à 10, présentant au moins une, de préférence au moins deux, de préférence au moins trois, de préférence au moins quatre, de préférence toutes les caractéristiques suivantes :
• R¹" est choisi parmi l'atome d'hydrogène, un groupement hydrocarboné linéaire ou ramifié comprenant de 1 à 12 atomes de carbone, et un groupement -(CH₂)_{p"}-S-C(X"Y")-(CH₂)_{n"}-C(O)OR³" ;
• R²" est choisi parmi un groupement hydrocarboné linéaire ou ramifié comprenant de 1 à 12 atomes de carbone, et un groupement -(CH₂)_{p"}-S-C(X"Y")-(CH₂)_{n"}-C(O)OR³" ;
• R³" représente un groupement hydrocarboné linéaire ou ramifié comprenant de 1 à 6 atomes de carbone ;
• R⁴" est choisi parmi l'atome d'hydrogène et un groupement hydrocarboné linéaire ou ramifié comprenant de 1 à 6 atomes de carbone ;
• T" est choisi parmi une liaison simple, -Sᵥ-, -(CH₂)_{t"}- et -C(CH₃)₂- ;
• X" et Y", indépendamment l'un de l'autre, représentent chacun un radical choisi parmi l'atome d'hydrogène et les radicaux alkyle, linéaires ou ramifiés, comportant de 1 à 6 atomes de carbone ;
• t" représente un entier compris entre 1 et 3, bornes incluses ;
• v" représente un entier compris entre 1 et 6, bornes incluses ;
• m" représente 0, ou un entier compris entre 1 et 10, bornes incluses ;
• n" représente un entier compris entre 8 et 20, bornes incluses ;
• p" représente 2 ;
• étant entendu qu'au moins un des groupements R¹" ou R²" représente un groupement -(CH₂)_{p"}-S-C(X"Y)"-(CH₂)_{n"}-C(O)OR³", où R³", X", Y", n" et p" sont tels que définis ci-dessus.

12. Composé selon l'une quelconque des revendications 8 à 11, présentant au moins une, de préférence au moins deux, de préférence au moins trois, de préférence au moins quatre, de préférence toutes les caractéristiques suivantes :
• R¹" est choisi parmi l'atome d'hydrogène, et un groupement hydrocarboné linéaire ou ramifié comprenant de 1 à 6 atomes de carbone ;
• R²" représente un groupement -(CH₂)_{p"}-S-C(X"Y")-(CH₂)_{n"}-C(O)OR³" ;
• R³" représente méthyle ou éthyle ;
• R⁴" représente l'atome d'hydrogène ;
• T" est choisi parmi une liaison simple, -Sᵥ-, -(CH₂)_{t"}- et -C(CH₃)₂- ;
• X" représente un radical choisi parmi l'atome d'hydrogène et les radicaux méthyle, éthyle, propyles et butyles ;
• Y" représente l'atome d'hydrogène ;
• t" représente un entier compris entre 1 et 3, bornes incluses ;
• v" représente un entier compris entre 1 et 6, bornes incluses ;
• m" représente 0, ou un entier compris entre 1 et 6, bornes incluses ;
• n" représente un entier compris entre 8 et 12, bornes incluses ; et
• p" est égal à 1.

13. Utilisation d'au moins un composé phénolique soufré de formule (1") selon l'une quelconque des revendications 8 à 12, comme anti-oxydant, stabilisant U.V., stabilisant thermique.

14. Utilisation selon la revendication 13, dans la préparation de matières plastiques, fibres synthétiques, élastomères, adhésifs, additifs et lubrifiants.

## Patentansprüche

1. Verfahren zur Herstellung von schwefelhaltigen Phenolverbindungen der folgenden Formel (1): 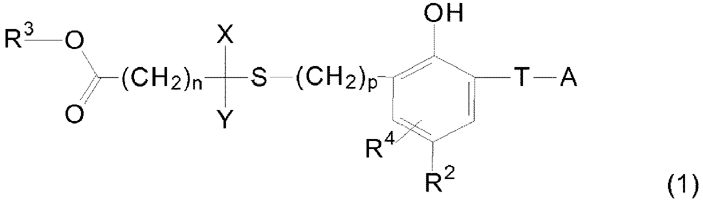 in der:
• A für einen Rest R¹ oder einen Rest der Formel A^{m}: 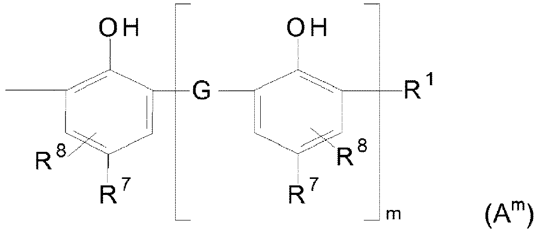 steht;
• R¹ aus einem Wasserstoffatom, einer linearen oder verzweigten Kohlenwasserstoffgruppe mit 1 bis 20 Kohlenstoffatomen und einer Gruppe -(CH₂)ₚ-S-C(XY)-(CH₂)ₙ-C(O)OR³ ausgewählt ist;
• R² aus einer linearen oder verzweigten Kohlenwasserstoffgruppe mit 1 bis 20 Kohlenstoffatomen und einer Gruppe - (CH₂)ₚ-S-C(XY)-(CH₂)ₙ-C(O)OR³ ausgewählt ist;
• R³ für eine lineare oder verzweigte Kohlenwasserstoffgruppe mit 1 bis 20 Kohlenstoffatomen steht;
• R⁴, R⁷ und R⁸ gleich oder verschieden sind und unabhängig voneinander aus einem Wasserstoffatom, einer linearen oder verzweigten Kohlenwasserstoffgruppe mit 1 bis 20 Kohlenstoffatomen und einer Gruppe -(CH₂)ₚ-S-C(XY)-(CH₂)ₙ-C(O)OR³ ausgewählt sind;
• G aus -S_{c}-, -(CH₂)ₐ-, -C(CH₃)₂-, -[S(O)_{b}]_{c}- und -W- ausgewählt ist, wobei W für eine gegebenenfalls durch eine oder mehrere Alkylgruppen substituierte aromatische Gruppe steht;
• T aus einer Einfachbindung, -Sᵥ-, -(CH₂)ₜ-, -C(CH₃)₂- und -[S(O)ᵤ]ᵥ- ausgewählt ist;
• X und Y unabhängig voneinander jeweils für einen Rest stehen, der aus einem Wasserstoffatom und linearen oder verzweigten Kohlenwasserstoffgruppen mit 1 bis 20 Kohlenstoffatomen und gegebenenfalls einem oder mehreren Heteroatomen, die aus Sauerstoff, Stickstoff und Schwefel ausgewählt sind, ausgewählt ist;
• a und t gleich oder verschieden sind und unabhängig voneinander jeweils für eine ganze Zahl zwischen 1 und 9, vorzugsweise zwischen 1 und 3, einschließlich der Grenzwerte stehen;
• b und u gleich oder verschieden sind und unabhängig voneinander jeweils für eine ganze Zahl mit einem Wert von 1 oder 2 stehen;
• c und v gleich oder verschieden sind und unabhängig voneinander jeweils für eine ganze Zahl zwischen 1 und 6 einschließlich der Grenzwerte stehen;
• m für 0 oder eine ganze Zahl zwischen 1 und 20 einschließlich der Grenzwerte steht;
• n für eine ganze Zahl zwischen 8 und 20 einschließlich der Grenzwerte steht;
• p für eine ganze Zahl zwischen 1 und 10 einschließlich der Grenzwerte steht;
• mit der Maßgabe, dass mindestens eine der Gruppen R¹, R² oder R⁴ für eine Gruppe -(CH₂)ₚ-S-C(XY)-(CH₂)ₙ-C(O)OR³ steht, wobei R³, X, Y, n und p wie oben definiert sind,
aus Ausgangsstoffen erneuerbaren Ursprungs, wobei das Verfahren mindestens die folgenden Schritte a) bis c) umfasst:
a) Umsetzung eines Mercaptoalkanoats der Formel (2) : 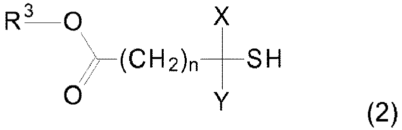 in der R³, X, Y und n wie oben definiert sind, mit einer Aminverbindung der Formel (3): 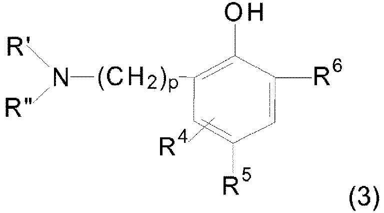 in der R⁴ und p wie oben definiert sind und
• R' und R" gleich oder verschieden sind und unabhängig voneinander aus einem Wasserstoffatom und einem linearen oder verzweigten Alkylrest mit 1 bis 6 Kohlenstoffatomen ausgewählt sind oder zusammen mit dem Stickstoffatom, das sie trägt, einen Heterocyclus bilden,
• R⁵ für eine lineare oder verzweigte Kohlenwasserstoffgruppe mit 1 bis 20 Kohlenstoffatomen oder eine Gruppe -(CH₂)ₚ-NR'R" steht und
• R⁶ aus einem Wasserstoffatom, einer linearen oder verzweigten Kohlenwasserstoffgruppe mit 1 bis 20 Kohlenstoffatomen und einer Gruppe -(CH₂)ₚ-NR'R" ausgewählt ist,
b) gegebenenfalls Umsetzung der in Schritt a) erhaltenen Verbindung mit einer Verbindung der Formel (4): 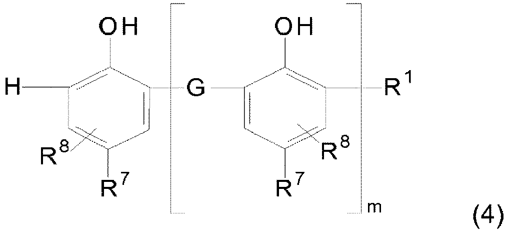 in der R¹, R⁷, R⁸, m und G wie oben definiert sind, über eine aromatische elektrophile Substitutionsreaktion und
c) Extraktion und dann gegebenenfalls Reinigung der Verbindung der Formel (1).

2. Verfahren nach Anspruch 1, bei dem die Reste X und Y der Verbindung der Formel (1) jeweils für ein Wasserstoffatom stehen.

3. Verfahren nach Anspruch 1 oder Anspruch 2, bei dem der Rest R⁴ der Verbindung der Formel (1) für ein Wasserstoffatom steht.

4. Verfahren nach einem der vorhergehenden Ansprüche, bei dem die Verbindung der Formel (1) mindestens ein, vorzugsweise mindestens zwei, vorzugsweise mindestens drei, vorzugsweise mindestens vier und vorzugsweise mindestens alle der folgenden Merkmale aufweist:
• R¹ ist aus einem Wasserstoffatom und einer linearen oder verzweigten Kohlenwasserstoffgruppe mit 1 bis 6 Kohlenstoffatomen ausgewählt;
• R² steht für eine Gruppe -(CH₂)ₚ-S-C(XY)-(CH₂)ₙ-C(O)OR³;
• R³ steht für Methyl oder Ethyl;
• R⁴ steht für ein Wasserstoffatom;
• T ist aus einer Einfachbindung, -Sᵥ-, -(CH₂)ₜ- und -C(CH₃)₂- ausgewählt;
• X steht für einen Rest, der aus einem Wasserstoffatom und Methyl-, Ethyl-, Propyl- und Butylresten ausgewählt ist;
• Y steht für ein Wasserstoffatom;
• t steht für eine ganze Zahl zwischen 1 und 3 einschließlich der Grenzwerte;
• v steht für eine ganze Zahl zwischen 1 und 6 einschließlich der Grenzwerte;
• m steht für 0 oder eine ganze Zahl zwischen 1 und 6 einschließlich der Grenzwerte;
• n steht eine ganze Zahl zwischen 8 und 12 einschließlich der Grenzwerte; und
• p ist gleich 1.

5. Verfahren nach einem der vorhergehenden Ansprüche, bei dem die Verbindung der Formel (4) aus Phenol/Aldehyd-Harzen, schwefelhaltigen Phenolharzen und Phenolharzen mit gem-Dimethylbrücken, die sich aus der Oligomerisationsreaktion von ortho-Isopropenyl-para-alkylphenol ergeben, ausgewählt wird.

6. Verfahren nach einem der vorhergehenden Ansprüche, das mindestens folgende Schritte umfasst:
a0) Bereitstellung mindestens eines Triglycerids;
a1) Umesterung des mindestens einen Triglycerids in Gegenwart eines Alkohols und Entfernung des gebildeten Glycerins zum Erhalt eines ungesättigten Esters;
a1') gegebenenfalls Behandlung des ungesättigten Esters durch Metathese oder Pyrolyse;
a2) Sulfhydrierung des ungesättigten Esters aus Schritt a1) bzw. a1') zum Erhalt des Mercaptoesters der Formel (2) gemäß obiger Definition;
a) Umsetzung des Mercaptoesters der Formel (2) gemäß der in Anspruch 1 angegebenen Definition mit einer amingruppenhaltigen Phenolverbindung der Formel (3) gemäß der in Anspruch 1 angegebenen Definition;
b) gegebenenfalls Umsetzung der in Schritt a) erhaltenen Verbindung mit einer Verbindung der Formel (4) gemäß der in Anspruch 1 angegebenen Definition, gegebenenfalls in Gegenwart eines Reagens mit einer Gruppe T gemäß der in Anspruch 1 angegebenen Definition;
c) Extraktion und dann gegebenenfalls Reinigung der in Schritt b) erhaltenen Verbindungen.

7. Verfahren nach einem der vorhergehenden Ansprüche, bei dem es sich bei dem Ausgangsstoff erneuerbaren Ursprungs um ein Triglycerid aus tierischen oder pflanzlichen, vorzugsweise pflanzlichen, Ölen oder Fetten, die aus Sojaöl, Sonnenblumenöl, Leinöl, Colzaöl, Rizinusöl, Palmöl, Palmkernöl, Kokosnussöl, Jatrophaöl, Baumwollsaatöl, Erdnussöl, Olivenöl, Vernoniaöl, Cupheaöl, Heveaöl, Lunariaöl, Saffloröl, Dotteröl, *Calophyllum-inophyllum-*Öl*, Pongamia-pinnata*-Öl, Rindertalg, Speiseöl oder Fett ausgewählt sind, bei denen es sich aber auch um Hydraulik- oder Schmieröle handeln kann, handelt.

8. Verbindung der Formel (1"): 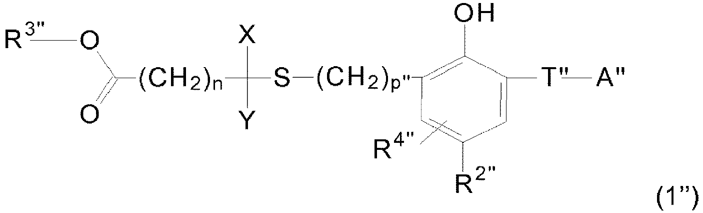 in der:
• A" für einen Rest R¹" oder einen Rest der Formel A^{m}": 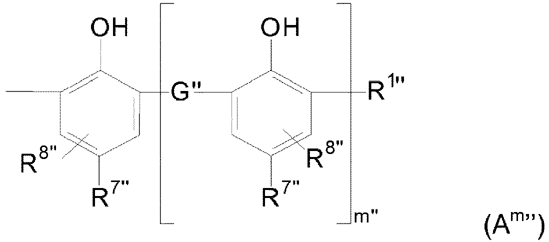 steht;
• R¹" aus einem Wasserstoffatom, einer linearen oder verzweigten Kohlenwasserstoffgruppe mit 1 bis 20 Kohlenstoffatomen und einer Gruppe -(CH₂)_{p"}-S-C(X"Y")-(CH₂)_{n"}-C(O)OR³" ausgewählt ist;
• R²" aus einer linearen oder verzweigten Kohlenwasserstoffgruppe mit 1 bis 20 Kohlenstoffatomen und einer Gruppe -(CH₂)_{p"}-S-C(X"Y")-(CH₂)_{n"}-C(O)OR³" ausgewählt ist;
• R³" für eine lineare oder verzweigte Kohlenwasserstoffgruppe mit 1 bis 20 Kohlenstoffatomen steht;
• R⁴", R⁷" und R⁸" gleich oder verschieden sind und unabhängig voneinander aus einem Wasserstoffatom, einer linearen oder verzweigten Kohlenwasserstoffgruppe mit 1 bis 20 Kohlenstoffatomen und einer Gruppe -(CH₂)_{p"}-S-C(X"Y")-(CH₂)_{n"}-C(O)OR³" ausgewählt sind;
• G" aus -S_{c"}-, - (CH₂)_{a"}-, -C(CH₃)₂-, -[S(O)_{b"}]_{c"} - und -W"- ausgewählt ist, wobei W" für eine gegebenenfalls durch eine oder mehrere Alkylgruppen substituierte aromatische Gruppe steht;
• T" aus einer Einfachbindung, -S_{v"}-, -(CH₂)_{t"}-, -C(CH₃)₂- und -[S(O)_{u"}]_{v"} - ausgewählt ist;
• X" und Y" unabhängig voneinander jeweils für einen Rest stehen, der aus einem Wasserstoffatom und linearen oder verzweigten Kohlenwasserstoffgruppen mit 1 bis 20 Kohlenstoffatomen und gegebenenfalls einem oder mehreren Heteroatomen, die aus Sauerstoff, Stickstoff und Schwefel ausgewählt sind, ausgewählt ist;
• a" und t" gleich oder verschieden sind und unabhängig voneinander jeweils für eine ganze Zahl zwischen 1 und 9, vorzugsweise zwischen 1 und 3, einschließlich der Grenzwerte stehen;
• b" und u" gleich oder verschieden sind und unabhängig voneinander jeweils für eine ganze Zahl mit einem Wert von 1 oder 2 stehen;
• c" und v" gleich oder verschieden sind und unabhängig voneinander jeweils für eine ganze Zahl zwischen 1 und 6 einschließlich der Grenzwerte stehen;
• m" für 0 oder eine ganze Zahl zwischen 1 und 20 einschließlich der Grenzwerte steht;
• n" für eine ganze Zahl zwischen 8 und 20 einschließlich der Grenzwerte steht;
• p" für eine ganze Zahl zwischen 1 und 10 einschließlich der Grenzwerte steht; und
• mit der Maßgabe, dass mindestens eine der Gruppen R¹", R²" oder R⁴" für eine Gruppe - (CH₂)_{p"}-S-C(X"Y")-(CH₂)ₙ-C(O)OR³" steht, wobei R³", X", Y", n" und p'' wie oben definiert sind und
• mit der Maßgabe, dass dann, wenn p" = 1, A" für einen Rest der Formel A^{m}" steht.

9. Verbindung nach Anspruch 8, wobei die Reste X" und Y" jeweils für ein Wasserstoffatom stehen.

10. Verbindung nach Anspruch 8 oder Anspruch 9, wobei der Rest R⁴" für ein Wasserstoffatom steht.

11. Verbindung nach einem der Ansprüche 8 bis 10, die mindestens ein, vorzugsweise mindestens zwei, vorzugsweise mindestens drei, vorzugsweise mindestens vier und vorzugsweise mindestens alle der folgenden Merkmale aufweist:
• R¹" ist aus einem Wasserstoffatom, einer linearen oder verzweigten Kohlenwasserstoffgruppe mit 1 bis 12 Kohlenstoffatomen und einer Gruppe -(CH₂)_{p"}-S-C(X"Y")-(CH₂)_{n"}-C(O)OR³" ausgewählt;
• R²" ist aus einer linearen oder verzweigten Kohlenwasserstoffgruppe mit 1 bis 12 Kohlenstoffatomen und einer Gruppe -(CH₂)_{p"}-S-C(X"Y")-(CH₂)_{n"}-C(O)OR³" ausgewählt;
• R³" steht für eine lineare oder verzweigte Kohlenwasserstoffgruppe mit 1 bis 6 Kohlenstoffatomen;
• R⁴" ist aus einem Wasserstoffatom und einer linearen oder verzweigten Kohlenwasserstoffgruppe mit 1 bis 6 Kohlenstoffatomen ausgewählt;
• T" ist aus einer Einfachbindung, -S_{v"}-, -(CH₂)_{t"}- und -C(CH₃)₂- ausgewählt;
• X" und Y" stehen unabhängig voneinander jeweils für einen Rest, der aus einem Wasserstoffatom und linearen oder verzweigten Alkylresten mit 1 bis 6 Kohlenstoffatomen ausgewählt ist;
• t" steht für eine ganze Zahl zwischen 1 und 3 einschließlich der Grenzwerte;
• v" steht für eine ganze Zahl zwischen 1 und 6 einschließlich der Grenzwerte;
• m" steht für 0 oder eine ganze Zahl zwischen 1 und 10 einschließlich der Grenzwerte;
• n" steht eine ganze Zahl zwischen 8 und 20 einschließlich der Grenzwerte; und
• p" steht für 2;
• mit der Maßgabe, dass mindestens eine der Gruppen R¹" oder R²" für eine Gruppe -(CH₂)_{p"}-S-C(X"Y")-(CH₂)_{n"}-C(O)OR³" steht, wobei R³", X", Y", n" und p" wie oben definiert sind.

12. Verbindung nach einem der Ansprüche 8 bis 11, die mindestens ein, vorzugsweise mindestens zwei, vorzugsweise mindestens drei, vorzugsweise mindestens vier und vorzugsweise mindestens alle der folgenden Merkmale aufweist:
• R¹" ist aus einem Wasserstoffatom und einer linearen oder verzweigten Kohlenwasserstoffgruppe mit 1 bis 6 Kohlenstoffatomen ausgewählt;
• R²" steht für eine Gruppe -(CH₂)_{p"}-S-C(X"Y")-(CH₂)_{n"}-C(O)OR³";
• R³" steht für Methyl oder Ethyl;
• R⁴" steht für ein Wasserstoffatom;
• T" ist aus einer Einfachbindung, -S_{v"}-, -(CH₂)_{t"}- und -C(CH₃)₂- ausgewählt;
• X" steht für einen Rest, der aus einem Wasserstoffatom und Methyl-, Ethyl-, Propyl- und Butylresten ausgewählt ist;
• Y" steht für ein Wasserstoffatom;
• t" steht für eine ganze Zahl zwischen 1 und 3 einschließlich der Grenzwerte;
• v" steht für eine ganze Zahl zwischen 1 und 6 einschließlich der Grenzwerte;
• m" steht für 0 oder eine ganze Zahl zwischen 1 und 6 einschließlich der Grenzwerte;
• n" steht eine ganze Zahl zwischen 8 und 12 einschließlich der Grenzwerte; und
• p" ist gleich 1.

13. Verwendung mindestens einer schwefelhaltigen Phenolverbindung der Formel (1") nach einem der Ansprüche 8 bis 12 als Antioxidans, UV-Stabilisator oder Wärmestabilisator.

14. Verwendung nach Anspruch 13 bei der Herstellung von Kunststoffen, Synthesefasern, Elastomeren, Klebstoffen, Additiven und Schmiermitteln.

## Claims

1. Process for preparing, from raw materials of renewable origin, sulfureous phenolic compounds corresponding to formula (1) below: 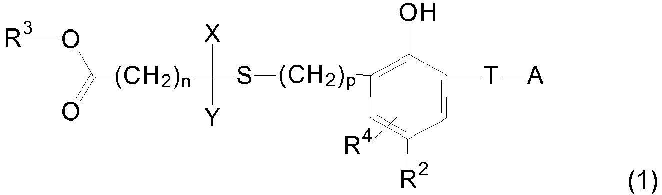 in which:
• A represents a radical R¹ or a radical of formula A^{m}: 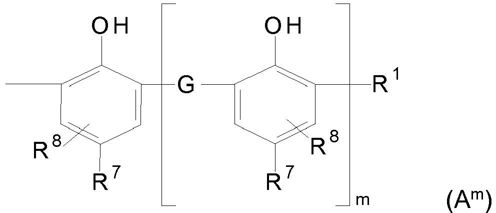
• R¹ is chosen from a hydrogen atom, a linear or branched hydrocarbon-based group comprising from 1 to 20 carbon atoms and a group -(CH₂)ₚ-S-C(XY)-(CH₂)ₙ-C(O)OR³;
• R² is chosen from a linear or branched hydrocarbon-based group comprising from 1 to 20 carbon atoms and a group -(CH₂)ₚ-S-C(XY)-(CH₂)ₙ-C(O)OR³;
• R³ represents a linear or branched hydrocarbon-based group comprising from 1 to 20 carbon atoms;
• R⁴, R⁷ and R⁸, which may be identical or different, are chosen, independently of each other, from a hydrogen atom, a linear or branched hydrocarbon-based group comprising from 1 to 20 carbon atoms and a group -(CH₂)ₚ-S-C(XY)-(CH₂)ₙ-C(O)OR³;
• G is chosen from -S_{c}-, -(CH₂)ₐ-, -C(CH₃)₂-, -[S(O)_{b}]_{c}- and -W-, in which W is an aromatic group, optionally substituted with one or more alkyl groups;
• T is chosen from a single bond, -Sᵥ-, -(CH₂)ₜ-, -C(CH₃)₂-, and -[S(O)ᵤ]ᵥ-;
• X and Y, independently of each other, each represent a radical chosen from a hydrogen atom and linear or branched hydrocarbon-based groups, comprising from 1 to 20 carbon atoms, and optionally comprising one or more heteroatoms chosen from oxygen, nitrogen and sulfur;
• a and t, which may be identical or different and independently of each other, each represent an integer between 1 and 9 and preferably between 1 and 3, limits inclusive;
• b and u, which may be identical or different and independently of each other, each represent an integer equal to 1 or 2;
• c and v, which may be identical or different and independently of each other, each represent an integer between 1 and 6, limits inclusive;
• m represents 0, or an integer between 1 and 20, limits inclusive;
• n represents an integer between 8 and 20, limits inclusive;
• p represents an integer between 1 and 10, limits inclusive;
• it being understood that at least one of the groups R¹, R² or R⁴ represents a group -(CH₂)ₚ-S-C(XY)-(CH₂)ₙ-C(O)OR³, in which R³, X, Y, n and p are as defined above,
said process comprising at least steps a) to c) below:
a) reaction of a mercapto alkoxide of formula (2): 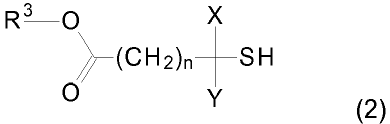 in which R³, X, Y and n are as defined previously, with an amine compound of formula (3): 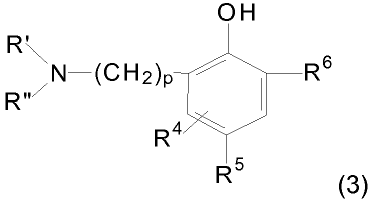 in which R⁴ and p are as defined previously and
• R' and R", which may be identical or different, are chosen, independently of each other, from a hydrogen atom, a linear or branched alkyl radical comprising from 1 to 6 carbon atoms, or form, together with the nitrogen atom that bears them, a heterocycle,
• R⁵ represents a linear or branched hydrocarbon-based group comprising from 1 to 20 carbon atoms or a group -(CH₂)ₚ-NR'R", and
• R⁶ is chosen from a hydrogen atom, a linear or branched hydrocarbon-based group comprising from 1 to 20 carbon atoms, and a group -(CH₂)ₚ-NR'R",
b) optionally, reaction of the compound obtained in step a) with a compound of formula (4): 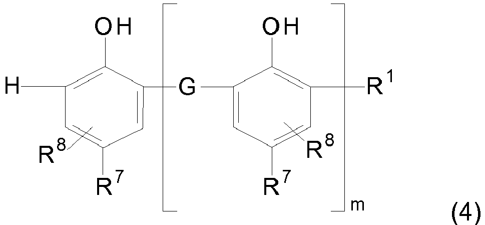 in which R¹, R⁷, R⁸, m and G are as defined previously,
via an aromatic electrophilic substitution reaction, and
c) extraction and then optional purification of the compound of formula (1).

2. Process according to Claim 1, in which the radicals X and Y of the compound of formula (1) each represent a hydrogen atom.

3. Process according to Claim 1 or Claim 2, in which the radical R⁴ of the compound of formula (1) represents a hydrogen atom.

4. Process according to any one of the preceding claims, in which the compound of formula (1) has at least one, preferably at least two, preferably at least three, preferably at least four, preferably all of the following characteristics:
• R¹ is chosen from a hydrogen atom and a linear or branched hydrocarbon-based group comprising from 1 to 6 carbon atoms;
• R² represents a group -(CH₂)ₚ-S-C(XY)-(CH₂)ₙ-C(O)OR³;
• R³ represents methyl or ethyl;
• R⁴ represents a hydrogen atom;
• T is chosen from a single bond, -Sᵥ-, -(CH₂)ₜ- and -C(CH₃)₂-;
• X represents a radical chosen from a hydrogen atom and methyl, ethyl, propyl and butyl radicals;
• Y represents a hydrogen atom;
• t represents an integer between 1 and 3, limits inclusive;
• v represents an integer between 1 and 6, limits inclusive;
• m represents 0, or an integer between 1 and 6, limits inclusive;
• n represents an integer between 8 and 12, limits inclusive; and
• p is equal to 1.

5. Process according to any one of the preceding claims, in which the compound of formula (4) is chosen from phenol/aldehyde resins, sulfureous phenolic resins and phenolic resins with gem-dimethyl bridges derived from the oligomerization reaction of ortho-isopropenyl-para-alkylphenol.

6. Process according to any one of the preceding claims, comprising at least the following steps:
a0) provision of at least one triglyceride;
a1) transesterification of said at least one triglyceride, in the presence of an alcohol, and removal of the glycerol formed, to obtain an unsaturated ester;
a1') optional treatment by metathesis or pyrolysis of said unsaturated ester;
a2) sulfhydration of the unsaturated ester from step a1) or a1') to obtain the mercapto ester of formula (2) as defined previously;
a) reaction of the mercapto ester of formula (2) as defined in Claim 1 with an amine phenolic compound of formula (3) as defined in Claim 1;
b) optionally, reaction of the compound obtained in step a) with a compound of formula (4) as defined in Claim 1, optionally in the presence of a reagent bearing the group T as defined in Claim 1;
c) extraction and then optional purification of the compounds obtained in step b).

7. Process according to any one of the preceding claims, in which the raw material of renewable origin is a triglyceride originating from animal or plant, preferably plant, oils or fats, chosen from soybean oil, sunflower oil, linseed oil, rapeseed oil, castor oil, palm oil, palm kernel oil, coconut oil, jatropha oil, cotton seed oil, groundnut oil, olive oil, vernonia oil, cuphea oil, hevea oil, lunaria oil, safflower oil, camellina oil, *Calophyllum inophyllum* oil, *Pongamia pinnata* oil, beef tallow, cooking oil or grease, but may also be hydraulic or lubricant oils.

8. Compound of formula (1"): 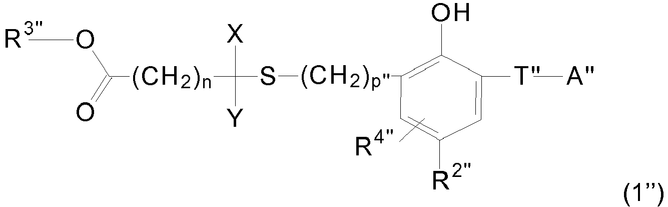 in which:
• A" represents a radical R¹" or a radical of formula A^{m}": 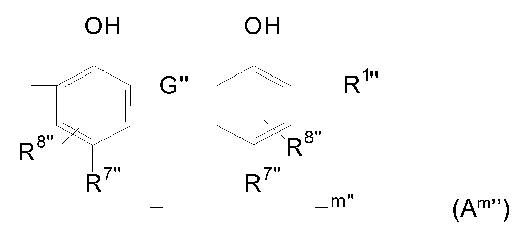
• R¹" is chosen from a hydrogen atom, a linear or branched hydrocarbon-based group comprising from 1 to 20 carbon atoms and a group -(CH₂)_{p"}-S-C(X"Y")-(CH₂)ₙ-C(O)OR³";
• R²" is chosen from a linear or branched hydrocarbon-based group comprising from 1 to 20 carbon atoms and a group -(CH₂)_{p"}-S-C(X"Y")-(CH₂)_{n"}-C(O)OR³";
• R³" represents a linear or branched hydrocarbon-based group comprising from 1 to 20 carbon atoms;
• R⁴", R⁷" and R⁸", which may be identical or different, are chosen, independently of each other, from a hydrogen atom, a linear or branched hydrocarbon-based group comprising from 1 to 20 carbon atoms and a group -(CH₂)_{p"}-S-C(X"Y")-(CH₂)_{n"}-C(O)OR³";
• G" is chosen from -S_{c"}-, -(CH₂)_{a"}-, -C(CH₃)₂-, -[S(O)_{b"}]_{c"}- and -W"-, in which W" is an aromatic group, optionally substituted with one or more alkyl groups;
• T" is chosen from a single bond, -S_{v"}-, -(CH₂)t_{"}-, -C(CH₃)₂- and -[S(O)_{u"}]_{v"}-;
• X" and Y", independently of each other, each represent a radical chosen from a hydrogen atom and linear or branched hydrocarbon-based groups, comprising from 1 to 20 carbon atoms, and optionally comprising one or more heteroatoms chosen from oxygen, nitrogen and sulfur;
• a" and t", which may be identical or different and independently of each other, each represent an integer between 1 and 9 and preferably between 1 and 3, limits inclusive;
• b" and u", which may be identical or different and independently of each other, each represent an integer equal to 1 or 2;
• c" and v", which may be identical or different and independently of each other, each represent an integer between 1 and 6, limits inclusive;
• m" represents 0, or an integer between 1 and 20, limits inclusive;
• n" represents an integer between 8 and 20, limits inclusive;
• p" represents an integer between 1 and 10, limits inclusive;
• it being understood that at least one of the groups R^{1"}, R^{2"} or R^{4"} represents a group -(CH₂)_{p"}-S-C(X"Y")-(CH₂)ₙ-C(O)OR^{3"}, in which R^{3"}, X", Y", n" and p" are as defined above, and
• it being understood that when p" = 1, then A" represents a radical of formula A^{m"}.

9. Compound according to Claim 8, in which the radicals X" and Y" each represent a hydrogen atom.

10. Compound according to Claim 8 or Claim 9, in which the radical R^{4"} represents a hydrogen atom.

11. Compound according to any one of Claims 8 to 10, having at least one, preferably at least two, preferably at least three, preferably at least four, preferably all of the following characteristics:
• R^{1"} is chosen from a hydrogen atom, a linear or branched hydrocarbon-based group comprising from 1 to 12 carbon atoms and a group -(CH₂)_{p"}-S-C(X"Y")-(CH₂)_{n"}-C(O)OR^{3"};
• R^{2"} is chosen from a linear or branched hydrocarbon-based group comprising from 1 to 12 carbon atoms and a group -(CH₂)_{p"}-S-C(X"Y")-(CH₂)_{n"}-C(O)OR^{3"};
• R^{3"} represents a linear or branched hydrocarbon-based group comprising from 1 to 6 carbon atoms;
• R^{4"} is chosen from a hydrogen atom and a linear or branched hydrocarbon-based group comprising from 1 to 6 carbon atoms;
• T" is chosen from a single bond, -S_{v"}-, -(CH₂)_{t"}- and -C(CH₃)₂-;
• X" and Y", independently of each other, each represent a radical chosen from a hydrogen atom and linear or branched alkyl radicals comprising from 1 to 6 carbon atoms;
• t" represents an integer between 1 and 3, limits inclusive;
• v" represents an integer between 1 and 6, limits inclusive;
• m" represents 0, or an integer between 1 and 10, limits inclusive;
• n" represents an integer between 8 and 20, limits inclusive;
• p" represents 2;
• it being understood that at least one of the groups R^{1"} or R^{2"} represents a group -(CH₂)_{p"}-S-C(X"Y")-(CH₂)_{n"}-C(O)OR^{3"}, in which R^{3"}, X", Y", n" and p" are as defined above.

12. Compound according to any one of Claims 8 to 11, having at least one, preferably at least two, preferably at least three, preferably at least four, preferably all of the following characteristics:
• R^{1"} is chosen from a hydrogen atom and a linear or branched hydrocarbon-based group comprising from 1 to 6 carbon atoms;
• R^{2"} represents a group -(CH₂)_{p"}-S-C(X"Y")-(CH₂)_{n"}-C(O)OR^{3"};
• R^{3"} represents methyl or ethyl;
• R^{4"} represents a hydrogen atom;
• T" is chosen from a single bond, -S_{v"}-, -(CH₂)_{t"}- and -C(CH₃)₂-;
• X" represents a radical chosen from a hydrogen atom and methyl, ethyl, propyl and butyl radicals;
• Y" represents a hydrogen atom;
• t" represents an integer between 1 and 3, limits inclusive;
• v" represents an integer between 1 and 6, limits inclusive;
• m" represents 0, or an integer between 1 and 6, limits inclusive;
• n" represents an integer between 8 and 12, limits inclusive; and
• p" is equal to 1.

13. Use of at least one sulfureous phenolic compound of formula (1") according to any one of Claims 8 to 12, as an antioxidant, UV stabilizer or heat stabilizer.

14. Use according to Claim 13, in the preparation of plastics, synthetic fibres, elastomers, adhesives, additives and lubricants.
